# EUROPÄISCHE PATENTANMELDUNG

(11) **EP 4 685 131 A1**
(43) Veröffentlichungstag der Anmeldung: **28.01.2026**
(21) Anmeldenummer: 24191095.9
(22) Anmeldetag: 26.07.2024
(51) Int. Cl.: C07C 209/68, C07C 211/36, B01J 8/02

(54) **VERFAHREN ZUR KONTINUIERLICHEN, KATALYTISCHEN HYDRIERUNG VON MDA**

(71) Anmelder: Evonik Operations GmbH, 45128 Essen (DE)
(72) Erfinder: KUHLMANN, Hanns, 45549 Sprockhövel (DE); RIX, Armin Matthias, 45770 Marl (DE); PAUL, Niklas, 45770 Marl (DE); WESSNER, Lea, 44139 Dortmund (DE); VATGAS GÓMEZ, Maria, 45721 Haltern am See (DE); WINKLER, Tobias, 48249 Dülmen (DE); BOECK, Florian, 58455 Witten (DE); SUDHOFF, Daniel, 59192 Bergkamen (DE); LETTMANN, Christian, 48653 Coesfeld (DE)
(74) Vertreter: Evonik Patent Association

(57) **Zusammenfassung**

Anlage zur Hydrierung von Methylendianilin (MDA; Edukt1) mit einem Wasserstoffgeber (Edukt2), insb. einem gasförmigen Wasserstoffgeber, vorzugsweises Wasserstoff (H2), umfassend eine Konditionierungseinheit für die Edukte, eine Reaktoreinheit zur Synthese von PACM und eine Trenneinheit, wobei
- die Konditionierungseinheit mind. eine Teillänge) der (Zu-)Leitungen für Edukte1, Edukt2 und mind. ein Lösungsmittel, mind. einen Wärmetauscher in mind. einer (Zu-)Leitung, mind. einen Mischer zur Mischung der Edukte und/oder mind. eines Edukts mit mind. einem Lösungsmittel umfasst;
- die Reaktoreinheit mind. einen Festbettreaktor als Hauptreaktor mit einer immobilen Katalysatorpackung umfasst, wobei der mind. eine (erste) Hauptreaktor
- einen ersten Strömungsweg für das Stoffgemisch
über die immobile Katalysatorpackung und
- einen weiteren hiervon getrennten, geschlossenen Strömungsweg für ein

Wärmetauschmittel außerhalb der Katalysatorpackung umfasst, und
wobei in den Medienumlauf ein Wärmetauscher eingebunden ist;
- die Trenneinheit mind.
- eine erste Trennstufe zur (im Wesentlichen) Abtrennung des Lösungsmittels und
- eine zweite Trennstufe zur (im Wesentlichen) Abtrennung vom Edukt und Nebenprodukten vom Produkt PACM umfasst, wobei die Trenneinheit in der ersten Trennstufe mind. ein Druckregeleinheit und einen Trennkessel mit einer Kondensationseinheit für das Lösungsmittel, wobei eine (Rück-)Leitung für das Lösungsmittel von der mind. einen Kondensationseinheit der ersten Trennstufe zur Konditionierungseinheit führt. Weiterhin ist von der Erfindung ein zugehöriges Verfahren umfasst.

## Beschreibung

Die Erfindung betrifft eine Anlage und ein Verfahren zur kontinuierlichen, katalytischen Hydrierung von MDA, insb. zur Herstellung von Methylenbis(cyclohexylamin), wie insb. 4,4'-Diaminodicyclohexylmethan (PACM).

Verfahren zur Hydrierung organischer Verbindungen, insbesondere zur Hydrierung von aromatischen Verbindungen zu den entsprechenden Cyclohexan-Derivaten sind aus dem Stand der Technik bereits bekannt.

Methylenbis(cyclohexylamin) ist ein bei Standardbedingungen (SATP) festes oder flüssig vorliegendes cycloaliphatisches Amin, das üblicherweise über die Flüssigphasen-Hydrierung von MDA hergestellt wird. Das Akronym MDA wurde historisch als Abkürzung für das bei der Umsetzung von Anilin und Formaldehyd gebildete, vor allem "Methylendianilin" (Diaminodiphenylmethan) umfassende Produktgemisch eingeführt und wird weiterhin zur Bezeichnung des mittlerweile großtechnisch erzeugten Verfahrensproduktes verwendet. Das Produkt der Hydrierung, das vor allem Methylenbis(cyclohexylamin) umfasst, wird deswegen oft auch als H12MDA bezeichnet.

MDA ist aufgrund seines Herstellungsprozesses üblicherweise ein Gemisch aus verschiedenen Diaminodiphenylmethanen. Vor allem besteht es aus 4,4`-Diaminodiphenylmethan. Es können jedoch auch 2,4`- und 2,2'-lsomere vorliegen. MDA kann weiterhin bei der Umsetzung von Anilin und Formaldehyd gebildete Reaktionsprodukte mit drei oder mehr aromatischen Ringen, insbesondere solche mit drei oder mehr Phenylringen, enthalten. Diese Reaktionsprodukte mit drei oder mehr aromatischen Ringen werden auch als Mehrkernverbindungen bezeichnet.

Bei dem im Handel erhältlichen Methylenbis(cyclohexylamin) handelt es sich aufgrund des hohen Anteils an 4,4`-Diaminodiphenylmethan im eingesetzten MDA größtenteils um 4,4'-Diaminodicyclohexylmethan bzw. Bis(para-Aminocyclohexyl)methan. Aufgrund der potentiellen Anwesenheit der entsprechenden 2,4'- und 2,2'-Diaminophenylmethan-lsomere im MDA kann in Methylenbis(cyclohexylamin) auch 2,4`-Diaminodicyclohexylmethan und 2,2'-Diaminodicyclohexylmethan vorliegen. Weiterhin kann hydriertes MDA neben Methylenbis(cyclohexylamin) weiterhin noch (ggf. partiell) hydrierte Mehrkernverbindungen enthalten.

US 5,578,546 A offenbart, dass 1947 erstmalig ein Verfahren zur Herstellung von Methylenbis(cyclohexylamin) beschrieben und 1965 in den technischen Maßstab überführt wurde. Die Hydrierung von MDA ist stark exotherm. So gibt WO 2010/069484 A1 eine Reaktionsenthalpie von -1600 kJ/mol an.

In Folge der Hydrierung werden je nach Verfahren verschiedene Diastereoisomere gebildet. Dabei kann das von 4,4`-Diaminodiphenylmethan abgeleitete Produkt 4,4'-Diaminodicyclohexylmethan (PACM) als trans/trans-, cis/cis- und cis/trans-Isomer vorliegen und ist deswegen in der Regel ein Gemisch dieser Isomere mit unterschiedlichen Anteilen. Mit steigendem trans/trans-Gehalt steigt dabei der Schmelzpunkt der Verbindung. Deshalb unterscheiden sich die Einsatzgebiete je nach Isomerengehalt stark: Während Methylenbis(cyclohexylamin)-Qualitäten mit niedrigem trans/trans - Gehalt (z.B. 10-30 Gew.-%) im Bereich der Amin- und Isocyanat-Vernetzer, insbesondere im Bereich von Zwei-Komponenten-Harzen Einsatz finden, finden Qualitäten mit hohem trans/trans - Gehalt (z.B. ≥ 48 Gew.-%) vor allem Anwendung als Regulator in Polyamidverbindungen. Gerade die Herstellung von Produkten mit niedrigem trans/trans-Gehalt stellt eine Herausforderung dar, da das thermodynamische Gleichgewicht, wie in US 3,636,108 A beschrieben wird, im Bereich wesentlich höherer trans/trans-Anteile (bis zu 51,2 %) liegt. US 2,606,925 A zeigt zudem, dass das Gleichgewicht nachträglich durch längeres Temperieren in Richtung eines höheren Anteils an trans/trans-Isomer verschoben werden kann.

Die Zusammensetzung des Hydrierungsproduktes hängt auch von der Zusammensetzung des eingesetzten MDAs ab: Oft wird MDA in Qualitäten von MDA50 bis MDA100 eingesetzt, wobei die zwischen 50 und 100 liegende Zahl den Gehalt an Diaminodiphenylmethanen im MDA-Gemisch angibt. MDA50 ist dabei eine MDA-Qualität, die wie oben erläutert prozessbedingt etwa 50 Gew.-% Diaminodiphenylmethanen und 50 Gew.-% Mehrkernverbindungen enthält. Die einzelnen Mehrkernverbindungen können entsprechend der enthaltenen Aromatenanzahl als 3-Kern-Verbindungen, 4-Kern-Verbindungen, etc. bezeichnet werden. MDA50 ist dabei die am meisten produzierte Qualität und wird hauptsächlich zu Methylendicyclohexyldiisocyanat (MDI) weiterverarbeitet. MDA100 ist reines MDA bzw. Diaminodiphenylmethan ohne Mehrkernverbindungen. MDA85 oder MDA90 sind andere auf dem Markt verfügbare Qualitäten mittlerer Reinheit. Wenn in Patentschriften zum Herstellverfahren von Methylenbis(cyclohexylamin) auf die Reinheit der MDA-Qualität eingegangen wird, so ist meist von MOA 100 die Rede (z.B. CN 110204447 B). Dagegen stellt US 2005/261525 A1 bevorzugt auf die Hydrierung von MDA50 ab. Die dabei als Hochsieder anfallenden hydrierten, oligomeren Amine eignen sich als Vernetzer mit besonders niedrigen Dampfdrücken für eine Reihe von Spezialanwendungen, wie US 2004/162409 A1 zeigt.

Der Gehalt an (ggf. partiell) hydrierten Mehrkernverbindungen im Produkt nimmt in der Reihenfolge der Edukte MDA50, MDA85, MDA90, MDA100 ab, da der Gehalt an Mehrkernverbindungen von MDA50 zu MDA100 abnimmt.

Aus der WO 2009/153123 A1 ist ein kontinuierliches Verfahren und einen Reaktor zur Hydrierung organischer Verbindungen in einem mehrphasigen, mehrstufigen System in Gegenwart eines homogenen oder heterogenen Katalysators bekannt. Als Katalysatoren werden u.a. Edelmetalle, wie Platin, Palladium, Ruthenium und Rhodium oder andere Übergangsmetalle, wie Molybdän, Wolfram und Chrom vorgeschlagen. Hierbei können die heterogenen Katalysatoren auf Trägermaterialien angeordnet sein, wie bspw. Kohlenstoff, Aluminiumoxid, Siliciumdioxid, Zirkoniumdioxid, Zeolithe, Alumosilikate oder Mischungen dieser Trägermaterialien. Als Substrate werden in diesem Verfahren bevorzugt aromatische Verbindungen enthaltend Amino-Substituenten eingesetzt, beispielsweise MDA, Polymer-MDA, Anilin, 2,4-Diaminotoluol, 2,6-Diaminotoluol, o-Phenylendiamin, etc. Die heterogenen Katalysatoren werden in Suspension eingesetzt.

DE 19533718 A1 offenbart ein Verfahren zur Hydrierung von aromatischen Verbindungen, in denen mindestens eine Aminogruppe an einen aromatischen Kern gebunden ist. Dazu kann ein heterogener Katalysator enthaltend Ruthenium und gegebenenfalls mindestens ein Metall der I-, VII- oder VIII-Nebengruppe verwendet werden. Als Trägermaterial wird beispielsweise Aluminiumoxid, Siliciumdioxid, Titandioxid oder Zirkoniumdioxid, vorzugsweise Aluminiumdioxid oder Zirkoniumdioxid, eingesetzt. Als Beispiel angegeben wird lediglich ein Katalysator enthaltend Ruthenium auf dem Trägermaterial Aluminiumoxid, nicht jedoch Zirkoniumoxid.

EP 1337331 A1 offenbart ein Verfahren zur katalytischen Hydrierung von aromatischen oder heteroaromatischen Aminen, wobei als Aktivmetall Ruthenium fungiert und der Katalysator mindestens ein weiteres Metall der I-, VII-, oder VIII-Nebengruppe enthält und diese auf einem Trägermaterial aufgebracht sind. Hierbei werden als aromatische Verbindungen u.a. 4,4'-MDA und Isomeren hiervon eingesetzt. Auch die EP 0111238 A1 offenbart ein Verfahren zur katalytischen Hydrierung von 4,4'-MDA, dadurch gekennzeichnet, dass die Hydrierung in Gegenwart von geträgertem Ruthenium in Anwesenheit von Nitraten und Sulfaten der Alkalimetalle und Nitraten der Erdalkalimetalle erfolgt. Ein vergleichbares Verfahren wird in der EP 1366812 A1 offenbart, wobei als Trägermaterialien unter anderem Aluminiumoxid, Siliciumoxid, Titanoxid und Zirkoniumoxid genannt werden.

Weitere Verfahren zur Hydrierung organischer Verbindungen offenbaren WO 2011/003899 A1 und WO 2009/090179 A1. Hier werden Verfahren zur Hydrierung von aromatischen Aminen mit Wasserstoff in Gegenwart eines Ru-Katalysators, welcher, u.a. Zirkoniumoxid-Trägermaterial enthält, offenbart.

Schließlich ist aus der EP 2 883 863 B1 ein Verfahren und eine Anlage zur Hydrierung von 4,4'-Methylendianilin (MDA) und/oder Polymer-MDA mit Wasserstoff in Gegenwarteines Katalysators bekannt. Hierbei wird als Katalysator Ruthenium vorgeschlagen, welcher auf einem Zirkoniumoxid-Trägermaterial aufgebracht ist. Bezüglich des Reaktors, verweist die EP 2 883 863 B1 auf den Reaktor bzw. das Reaktorkonzept der WO 2008/015135 A1. Aus dieser WO 2008/015135 A1 ist ein kontinuierliches Verfahren und eine Anlage zur Hydrierung von Diisononylphtalat zu 1,2-Cyclohexandicabonsäurediisononylester (DINCH) bekannt, wobei DINP als Gemisch in einem organischen Lösungsmittel, mit Wasserstoff bei einem Druck von bis zu 325 bar hydriert wird. Hierbei wird eine in Reihenschaltung von zwei Festbettreaktoren mit jeweils einer immobilen Festbettschüttung vorgeschlagen. Zur Ableitung der Reaktionswärme aus den beiden Reaktoren wird vorgeschlagen, einen Teilstrom des Stoffgemisches nach dem zweiten Festbettreaktor zu rezirkulieren und diesen hierbei zu kühlen. Ein vergleichbares Reaktorkonzept von in Reihe geschalteten Festbettreaktoren zur Hydrierung von ist auch aus der EP 1 566 372 B1 bekannt.

Nachteilig an dem Konzept ist, dass die Kreislaufführung eines Produktteilstroms zu einer erhöhten Bildung von unerwünschten Nebenprodukten führen kann und die Anlagenleistung senkt, wobei erhöhte Energiekosten für die Rückführung und Kühlung des Recyclingstroms erzeugt werden.

Wie ausgeführt, ist der Bedarf an bspw. PACM mit unterschiedlichen Anteilen der jeweiligen Isomeren abhängig vom dem Einsatzzweck bzw. den nachfolgenden Produkten. So sind beispielsweise PACM-Qualitäten mit niedrigem trans/trans-Gehalt von 10 bis 30 Gew.% im Bereich der Amin- und Isocyanat-Vernetzer bevorzugt, insbesondere im Bereich der Formulierung von 2-Komponenten-Harzen, wobei PACM-Qualitäten mit hohem trans/trans-Gehalt von über 48 Gew.% vor allem Anwendung als Regulator in Polyamidverbindungen finden, als Regulator in Polyamidverbindungen. Die genannten Gew.% beziehen sich hierbei auf das PACM-Isomerengemisch als solches. Gerade die Herstellung von Produkten mit niedrigem trans/trans-Gehalt stellt eine verfahrenstechnische Herausforderung dar, da das thermodynamische Gleichgewicht, wie in US 3,636,108 A beschrieben wird, im Bereich wesentlich höherer trans/trans-Anteile von bis zu 51,2 Gew.% liegt. Weiterhin ist aus der US 2,606,925 A bekannt, dass das Gleichgewicht der PACM-Isomere durch längeres Temperieren nachträglich in Richtung zu einem höheren Anteil an trans/trans-Isomer verschoben wird.

Aufgabe der vorliegenden Erfindung ist es somit, eine verbesserte Anlage und einen verbesserten Prozess bereitzustellen, der hinsichtlich Produktumsatz und Energieeffizienz verbessert ist und insb. die Herstellung definierter Anteile der jeweiligen Isomere im Isomerengemisch ermöglicht.

Die Aufgabe wird erfindungsgemäß mit einer Anlage nach den Merkmalen des Anspruch 1 und einem Verfahren nach den Merkmalen des Anspruchs 13 gelöst.

Hierbei wird eine Anlage vorgesehen, zur kontinuierlichen, katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2),
umfassend eine Konditionierungseinheit für die Edukte, eine Reaktoreinheit und eine Trenneinheit, wobei
- die Konditionierungseinheit mind. (Zu-)Leitungen für Edukte1, Edukt2 und mind. ein Lösungsmittel, mind. einen Wärmetauscher in mind. einer (Zu-)Leitung, mind. einer Mischereinheit zur Mischung der Edukte und/oder mind. eines Edukts mit mind. einem Lösungsmittel umfasst;
- die Reaktoreinheit mind. einen Festbettreaktor als Hauptreaktor mit einer immobilen Katalysatorpackung umfasst, wobei der mind. eine Hauptreaktor
   - einen ersten Strömungsweg für das Stoffgemisch über
      die immobile Katalysatorpackung und
   - einen weiteren hiervon getrennten, geschlossenen Strömungsweg, bzw. Medienumlauf, für ein Wärmetauschmittel außerhalb der Katalysatorpackung, d.h. dem ersten Strömungsweg, umfasst, und
wobei in den Medienumlauf ein Wärmetauscher eingebunden ist;
- die Trenneinheit mind.
- eine erste Trennstufe zur (im Wesentlichen) Abtrennung des Lösungsmittels und
- eine zweite Trennstufe zur (im Wesentlichen) Abtrennung vom mind. einem Edukt und/oder mind. einem Nebenprodukten vom Produkt umfasst, wobei die Trenneinheit in der ersten Trennstufe mind. eine Druckregeleinheit und einen Trennkessel mit einer Kondensationseinheit für das Lösungsmittel, wobei eine (Rück-)Leitung für das Lösungsmittel von der mind. einen Kondensationseinheit der ersten Trennstufe zur Konditionierungseinheit führt.

Die Anlage dient vorzugsweise zur kontinuierlichen, katalytischen Herstellung von Methylenbis(cyclohexylamin) als Produkt, insb. zur Produktion von 4,4'-Diaminodicyclohexylmethan (PACM), nach der Formel (I)

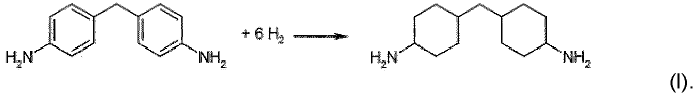

Der von dem Trennkessel kommende flüssige Stoffstrom wird über eine (Feed-)Leitung zur ersten Trennkolonne der ersten Trennstufe der Trenneinheit geleitet. Vorteilhafterweise kann bei einer Ausführungsform der Anlage vorgesehen sein, dass in der (Feed-)Leitung vom Trennkessel zur ersten Kolonne eine Druckregeleinheit vorgesehen ist. Hierdurch kann die Reaktoreinheit auf einem ersten, hohen Druckniveau betrieben werden und die erste Trennkolonne der ersten Trennstufe auf einem zweiten, tieferen Druckniveau, wobei der Trennkessel auf einem Zwischenniveau betrieben werden kann.

Weiterhin kann es vorteilhaft sein, wenn stromaufwärts zur ersten Trennkolonne der ersten Trennstufe ein (Vorlauf-)Wärmetauscher vorgesehen ist, insb. auch stromabwärts der Druckregeleinheit bzw. zwischen der Druckregeleinheit und der ersten Trennkolonne.

Bei einer besonders vorteilhaften Ausführungsform ist einen Energiekopplung vorgesehen, um den Kondensator stromabwärts zum Trennkessel, einen Kondensator einer Trennkolonne der zweiten Trennstufe oder den (Umlauf-)Wärmetauscher im Medienumlauf des mind. einen Hauptreaktors verschaltet im Wärmetausch mit dem (Vorlauf-)Wärmetauscher der ersten Trennkolonne zu betreiben. Die Energiekopplung kann durch Medienleitung und eine serielle Verschaltung der jeweiligen Wärmetauscher erfolgen oder durch eine integrierte Energiekopplung in einem (baulich) einzigen Wärmetauscher. Die integrierte Energiekopplung hat den Vorteil, sofern räumlich innerhalb der Anlage realisierbar, dass nur ein Temperaturgefälle für den Wärmetransport überwunden werden muss. Durch Energieübertrag kann so der (Feed-)Stoffstrom vor der ersten Trennkolonne der ersten Trennstufe, der nach der stromaufwärtsbefindlichen Druckregeleinheit in der (Feed-)Leitung ein Temperaturniveau von ca. 85 bis 95 °C aufweist, parallel angehoben werden. Da der Hauptreaktor im Laufe des Betriebes bei stetig steigender Temperatur betrieben wird, um die sinkende Katalysatoraktivität zu kompensieren, kann parallel eine stetig steigende Energiemenge an den (Feed-)Stoffstrom vorlaufend der ersten Trennkolonne abgegeben werden. Hierdurch sinkt ebenfalls stetig der Energiebedarf im Sumpfkreislauf, bzw. dem dortig eingebundenen Wärmetauscher der ersten Trennkolonne.

Mit dem separaten Nachreaktor, insb. dem adiabaten Nachreaktor, ist eine optimierte Steuerung des Prozesses und der Anlage möglich geworden, wodurch die Selektivität durch vom Hauptreaktor (Auslass) abweichende Eintrittstemperatur des Nachreaktors möglich wird. Typischerweise kann die Eintrittstemperatur des Nachreaktors bei der gleichen oder einer geringfügig niedrigeren Temperatur vorgesehen werden, die bis zu 30 °C unterhalb der Auslasstemperatur des Hauptreaktors liegen kann. Somit kann mind. zeitweise eine Temperaturerhöhung im (adiabatischen) Nachreaktor von 20 °C bis 40 °C, typischerweise von 20 °C bis 30 °C zugelassen werden, und so gezielt die gewünschte Produktqualität (Isomerenverhältnis) eingestellt werden. Auf diese Weise ist ein sehr niedriger und ein sehr genauer Anteil an trans/trans-Isomeren in dem Isomerengemisch einstellbar. Der Hauptreaktor kann auf einem möglichst niedrigen Temperaturniveau betrieben werden, so dass der trans/trans-Anteil des PACM im Stoffstrom (Auslass des Hauptreaktors) ca. 13 bis 20 Gew.% beträgt, wobei ca. 13 % bei einem neuen bzw. regenerierten Katalysator erreichbar sind und 20 Gew.% bei einem Katalysator nach Langzeitnutzung (kurz vor Austausch/Regeneration). Abhängig von der Phase, in der sich der Hauptreaktor befindet, kann es mind. zeitweise sinnvoll sein, wenn die Eintrittstemperatur des Nachreaktors um 5 bis 20 °C höher als die des Hauptreaktors ist.

Der mit Katalysator frisch befüllte oder regenerierte Hauptreaktor mit der dortigen stark exothermen Reaktion wird hierbei weitgehend isotherm betrieben, was aber nicht im idealen Sinne zu verstehen ist. Auch wenn der Hauptreaktor vorliegend als "isotherm/-isch" bezeichnet wird, wird dieser ideale Zustand bei industrieller Anwendung nur bedingt erreicht, so dass sich innerhalb des Hauptreaktors wegen unvollständigem Wärmeabtransport ein Temperaturgradient von ca. 5 bis 10 °C in radiale und auch in Strömungsrichtung ausbildet.

Der Nachreaktor wird über den vorlaufenden Wärmetauscher mit einer etwas höheren Temperatur derart beschickt, dass die gewünschte restliche Reaktion und Isomerenumformung zu dem finalen, gewünschten trans/trans-Verhältnis von bspw. 17 bis 23 Gew.% erfolgt. Der Nachreaktor mit der dortigen schwach exothermen, restlichen Reaktion wird hierbei weitgehend adiabat betrieben, was aber nicht im idealen Sinne zu verstehen ist.

Mit fortschreitender Zeit erfolgt ein Absinken der Katalysatoraktivität bis ein Austausch oder eine Regenerierung erforderlich ist. Hierzu wird parallel durch die Regelung des Kühlkreislaufs die Betriebstemperatur angehoben, d.h. insb. eine geringere Kühlung über den im Kühlkreislauf eingebundenen Wärmetauscher vorgenommen, um Umsatz und Selektivität insgesamt auf einem weitgehend gleichbleibenden Niveau zu halten. Dies hat zur Folge, dass das Isomerenverhältnis verschoben wird, hin zu höheren trans/trans-Anteilen im Produkt. Hierbei hat es sich als sehr vorteilhaft erwiesen, dass die Temperatur im Feed des Nachreaktors über den vorlaufenden Wärmetauscher eigenständig geregelt werden kann, insb. gleichläufig. So wird mit steigender Betriebstemperatur des Hauptreaktors über die Nutzungszeit des Katalysators die Feedtemperatur im Stoffstrom des Nachreaktors abgesenkt.

Der von einem Druckentspannungstrennkessel, nachstehend Trennkessel genannt (auch "Flashbehälter" genannt), kommende flüssige Stoffstrom wird über eine Leitung zur ersten Trennkolonne innerhalb der ersten Trennstufe der Trenneinheit geleitet. Der Trennkessel zeichnet sich dadurch aus, dass durch Entspannung (Druckabsenkung) der zufließende Stoffstrom in eine Dampfphase (Lösungsmittel, lösungsmittelreich) und eine Flüssigphase (verarmt an Lösungsmittel) aufgetrennt wird und im Regelbetrieb im Trennkessel beide Phasen vorliegen. Der Trennkessel kann zusätzlich einen Sumpfumlauf mit integriertem Wärmetauscher aufweisen oder hieran angeschlossen sein, um durch Erwärmung der Flüssigphase den abtrennbaren Dampfanteil über den druckbedingten Anteil hinaus zu erhöhen. Weiterhin kann ein Trennkessel Einbauten oder Füllkörper umfassen, insb. um das Mitreißen von nicht oder nur unvollständig an Lösungsmittel verarmten Tröpfchen zu verhindern. Vorteilhafterweise kann bei einer Ausführungsform der Anlage vorgesehen sein, dass in der Leitung vom Trennkessel zur ersten Kolonne eine Druckregeleinheit vorgesehen ist. Hierdurch kann die Reaktoreinheit auf einem ersten, hohen Druckniveau betrieben werden und die ersten Trennstufe der Trenneinheit auf einem zweiten, tieferen Druckniveau. Im vorliegenden Zusammenhang meint also Trennkessel (Flashbehälter) einen Apparat, indem im Wesentlichen durch Druckentspannung eine Phasentrennung veranlasst wird. Trennkolonne meint vorliegend hingegen einen Apparat, in dem im Wesentlichen durch Energiezufuhr einen Trennung in eine Dampfphase und Flüssigphase erfolgt, insb. unter Einbindung eines Kopfumlaufs, indem im Kopf mind. ein Teil der auskondensierten Flüssigkeit zurück in die Kolonne geleitet wird.

Vorliegend meint "Effizienzsteigerung", wenn nichts Abweichendes ausgeführt wird, einen geringeren Energieverbrauch. Der Bezug ergibt sich aus dem Zusammenhang und kann bezogen sein auf die Anlage, den jeweils beschriebenen Anlagenabschnitt oder den verbesserten Apparat bspw. durch Integration von zwei Wärmetauschern in einen einzigen.

Vorteilhafterweise ist die Mischereinheit zweiteilig ausgebildet und umfasst bspw. einen Mischapparat und einen Gas-Sättiger. Der Mischapparat kann insb. ein dynamischer oder statischer Mischer sein, der zur innigen Verbindung von MDA (Edukt1) mit dem Lösungsmittel geeignet ist. Der Gas-Sättiger kann insb. eine kleine Kolonne oder ein Kessel sein, sind dem geeignete Einbauten vorhanden sind, um das unter einem hohen Druck zugeführte H2-Gas, intensiv im MDA-Lösungsmittel-Stoffstrom zu lösen und/oder homogen vor dem Eintritt in den Hauptreaktor zu verteilen. Der H2-Gasdruck beträgt vorteilhafterweise 70 bar bis 100 bar.

Mit dem Begriff "immobile Katalysatorpackung" oder "immobiler Katalysator" ist jede Form eines lokalen, nicht mit dem Stoffstrom strömenden oder fließenden Katalysator gemeint, wie insb. Katalysatorschüttungen (Pellets oder beschichtete Trägerkörper) oder festen Einbauten, die mit Katalysatormaterial beschichtet sind. Vorteilhafte Einbauteil, die eine Katalysatorbeschichtung aufweisen, können bspw. Gittern, Platten oder sonstigen Köper sein, die im Hauptreaktor angeordnet sind.

Im vorliegenden Zusammenhang meint eine "XY-einheit" und/oder "XY-stufe" immer auch mind. einen entsprechenden Apparat, Vorrichtung oder dergleichen, der/die von der jeweiligen Einheit oder Stufe umfasst ist. So meint bspw. eine Mischeinheit/-stufe, dass diese mind. einen Mischer/- vorrichtung umfasst.

Im vorliegenden Zusammenhang meint "Wärmetausch" oder "Wärmetauscher" immer einen indirekten Wärmetausch und entsprechende Bauformen mit geschlossenen Stoff- und Medienführungen, es sei denn, es ist ausdrücklich etwas hiervon Abweichendes beschrieben.

Mit der "Kondensationseinheit" der ersten Trennstufe ist ein einzelner Wärmetauscher oder eine Gruppe von Wärmetauschern bezeichnet, die zur mind. teilweisen Kondensation und/oder Abkühlung der über die Kopfleitung/en abgeleiteten Anteile an Leichtsiedern dienen. Eine derart bezeichnete "Kondensationseinheit" muss keine (geschlossene) Baueinheit darstellen. Somit wird zum Teil der Begriff "Kondensationseinheit" und einen einzelner "Wärmetauscher" auch synonym verwendet.

Die als "erste Trennstufe" bezeichnete Stufe ist insb. dadurch bestimmt und weist entsprechende Apparate und Leitungen auf, dass das Lösungsmittel (gezielt) vom produktreichen Stoffstrom abgetrennt wird und vorteilhafterweise auch zur Verwendung in die Reaktoreinheit und/oder die Konditionierungseinheit zurückgeleitet wird. In analoger Weise meint die als "zweite Trennstufe" bezeichnete Stufe der Trenneinheit, dass diese dadurch bestimmt ist und entsprechende Apparate und Leitungsführungen aufweist, um das Produkt von Nebenprodukten und Edukten, insb. MDA zu trennen und das Produkt aufzureinigen. Hierbei sind die erste und zweite Stufe naturgemäß nicht ganz streng getrennt und es kann ein Überlappungsbereich oder ein Apparat im Übergangsbereich vorhanden sein, in dem sowohl Lösungsmittel als auch mind. ein Nebenprodukt oder mind. ein Edukt vom Produkt, insb. PACM, getrennt wird.

In dem vorliegenden Zusammenhang meint die "Abtrennung von Lösungsmittel" in der ersten Trennstufe und "Abtrennung von mind. einem Edukt und/oder mind. einem Nebenprodukt vom Produkt PACM", dass die Abtrennung keine absolute Abgrenzung der Trennstufen meint, sondern jeweils "im Wesentlichen" nur den/die genannten Stoff/e betrifft.

Der Begriff "Eduktmischung" meint in dem vorliegenden Zusammenhang die Mischung aus Stoffen, die beim Eintritt in den (ersten) Hauptreaktor vorliegt, also die Mischung aus allen Edukten, Lösungsmitteln, Hilfsstoffen etc. im und nach dem mind. einen Reaktor wird die strömende Mischung aus Stoffen in jedem Grad der Reaktion oder nachfolgenden Aufreinigung als "Stoffstrom" oder "Stoffmischung" (auch "Stoffgemisch") bezeichnet, wobei zum Teil adjektivische Beschreibungen wie "produktreich" oder "lösungsmittelreich" angefügt sind. Die stoffliche Zusammensetzung des Stoffstroms an jedem Ort der Anlage ergibt sich für den Fachmann allerdings auch in naheliegender Weise durch ebendiesen Anlagenort und stromaufwärts befindliche Anlagenkomponenten und insb. die verfahrenstechnischen Apparate der Anlagen. Die Reinstoffe, wie bspw. das Produkt PACM, sowie die Nebenprodukte LB und HB, sind gesondert benannt und ausgewiesen. Hierbei steht "LB" für "Low Boiler", einem werthaltigen Stoffgemisch, das gesondert aus dem Stoffstrom und vom Produkt abgetrennt wird und einen niedrigen Siedepunkt von ca. 240 °C bis 290 °C aufweist. In analoger Weise steht "HB" für "High Boiler", einem werthaltigen Stoffgemisch, das gesondert aus dem Stoffstrom und vom Produkt abgetrennt wird und einen hohen Siedepunkt von > 350 °C aufweist.

Vorliegend wird insb. eine Anlage und ein Verfahren zur Produktion von Methylenbis(cyclohexylamin) als Produkt, insb. zur Produktion von 4,4'-Diaminodicyclohexylmethan (PACM), durch katalytische Hydrierung von Methylendianilin beansprucht. Hierbei ist das primäre Produkt PACM, das aus 4,4`-Diaminodiphenylmethan (4,4`-MDA; primärer Anteil des Edukts1) hydriert wird, insb. mit dem genannten niedrigen trans/trans-Isomerenverhältnis, so dass die Anlage und das Verfahren insb. zur Produktion von 4,4`-Diaminodicyclohexylmethan (PACM) durch kontinuierliche, katalytische Hydrierung von 4,4'-Diaminodiphenylmethan (4,4'-MDA) dient und geeignet ist. Die weiterhin vorliegenden Anteile 2,4'-MDA und 2,2'-MDA des MDA werden mind. zu geringen Anteilen parallel zu Reaktionsprodukten gewandelt, wobei diese in der Regel in der Produktmischung verbleiben. Weiterhin können vorteilhafterweise ebenfalls durch dieses Verfahren bzw. diese Anlage in der zweite Trennstufe der Trenneinheit sonstige Nebenprodukte aufgereinigt und abgetrennt werden, insb. in mind. einer Trennkolonne. Diese stellen regelmäßig sekundäre (werthaltige) Produkte dar und sind hierin im Wesentlichen als High Boiler (HB) und Low Boiler (LB) beschrieben und gemeint.

Vorteilhafterweise ist das Lösungsmittel aus der nachfolgenden Gruppe von Stoffen: Cyclohexan, Dioxan, Tetrahydrofuran (THF), Cyclohexylamin, dicyclohexylamin, Methanol, Ethanol, Isopropanol, n-Butanol, 2-Butanol, 2-Methoxy-2-methylpropan (MTBE) oder Methylcyclohexan oder eine Gemisch hieraus. Vorteilhafterweise wird das Lösungsmittel, insb. THF im Überschuss zum MDA zugeführt, so dass vorteilhafterweise das Verhältnis der Massenströme von Lösungsmittel, insb. THF, zu MDA am Einlass des Hauptreaktors im Bereich von 1,0 bis 8,0 liegt, insbesondere im Bereich von 2,0 bis 6,0.Bei einer Ausführungsform der Anlage, kann es vorteilhaft sein, dass die Reaktoreinheit einen ersten Hauptreaktor und mind. einen stromabwärts befindlichen permanenten, in Serie geschalteten Nachreaktor umfasst. Der besondere Vorteil des Nachreaktors und dessen eingangsseitigen Temperierung des Stoffstroms besteht darin, dass auf diese Weise eine optimierte Steuerung der Selektivität der Anteile an Isomeren ermöglicht wird, aufgrund der vom ersten Hauptreaktor abweichenden, bevorzugt höheren Eintrittstemperatur. Vorteilhafterweise ist der Nachreaktor auch ein Festbettreaktor, bzw. Reaktor mit einem immobilen Katalysator, wie beispielsweise einer Katalysatorschüttung oder mit Katalysator beschichteten Einbauten.

Vorteilhafterweise umfasst der immobile Katalysator Ruthenium, ist mit Ruthenium dotiert oder ist hieraus gebildet. Bei einer vorteilhaften Verfahrensvariante, insb. zur Erreichung eines niedrigen Anteils an trans/trans Isomeren im Isomerengemisch, wird der Hauptreaktor bei einer Temperatur von 90 bis 140 °C, idealerweise 95 bis 135 °C.

Es hat sich als besonders vorteilhaft herausgestellt, wenn das Verhältnis der Katalysatormassen vom Hauptreaktor zum Nachreaktor ist im Bereich von 1,2 bis 2 liegt, bevorzugt 1,3 zu 1,4 und idealerweise 1,35. Es hat sich überraschenderweise gezeigt, dass es hinreichend ist, die stark exotherme Reaktion beim den Reaktionsstart im Hauptreaktor durch einen intensiven Kühlkreislauf zu regeln, und lediglich die Vorlauftemperatur im Zulauf zum Nachreaktor so einzustellen, dass der moderate Temperaturanstieg von ca. 30 bis 35 °C im Nachreaktor vom Einlass zum Auslass nur noch einen limitierten und gut reproduzierbaren Einfluss auf den trans/trans-Isomerenanteil im Stoffstrom bzw. im Produkt hat, wie bereits ausgeführt.

Bei einer weiteren Ausführungsform der Anlage, kann es vorteilhaft sein, dass
die Reaktoreinheit einen weiteren Hauptreaktor als Festbettreaktor umfasst, der
   - einen ersten Strömungsweg für das Stoffgemisch und
   - einen weiteren (geschlossenen) Strömungsweg für ein Wärmetauschmittel umfasst, und wobei stromaufwärts zu den beiden Hauptreaktoren in der (Zu-)Leitung eine Ventileinheit vorgesehen ist, mittels welcher der Volumenstrom der Eduktmischung zw. dem ersten Hauptreaktor und dem weiteren Hauptreaktor aufteilbar, durchleitbar und/oder vollständig umschaltbar ist, und wobei beide Hauptreaktoren
   - jeweils mit einem Wärmetauscher oder
   - einem gemeinsam mit einem Wärmetauscher
für den weiteren (geschlossenen) Strömungsweg verbunden sind.

Hierbei sind die beiden in Reihe geschalteten Hauptreaktoren baugleich oder im Wesentlichen baugleich. Insbesondere sind beide Hauptreaktoren derart dimensioniert und/oder weisen entsprechende Einbauten auf, dass dieselbe bzw. die im Wesentliche gleiche Masse und/oder Volumen an Katalysator angeordnet ist oder aufnehmbar ist.

Vorteil dieser beiden Hauptreaktoren liegt darin, dass eine weitere thermische Entkopplung der ersten Reaktionsphase mit sehr starker Exothermie, der zweite Reaktionsphase mit mittlerer Exothermie und der Nachreaktion mit sehr geringer Exothermie erfolgen kann. Weiter Vorteile bestehen darin, dass bei derselben Anlagenleistung, der einzelne Hauptreaktor kleiner dimensioniert ist und somit thermisch leichter und homogener geregelt werden kann. Zeitgleich wird die Anlagenleistung erhöht, weil im Wartungsfall, wie bspw. einem Katalysatorwechsel, die Anlage nicht vollständig stillgestellt werden muss. Hierzu sind die beiden Hauptreaktoren derart leitungstechnisch verschaltet, dass jeder auch alleinig vom Stoffgemisch durchströmbar ist, unter Umgehung des jeweils andere Hauptreaktors (Bypass 1).

Durch die Hydrierung im (isothermischen) Hauptreaktor mit starker Kühlung, kann die durch die Temperatur induzierte Isomerisierung stark begrenzt werden. Im (adiabatischen) Nachreaktor wird anschließend gezielt so viel Temperatur insb. durch Wärmetausch im zufließenden Stoffstrom eingeregelt und damit zugelassen, dass die Isomerisierung gerade einen spezifikationsgerechten trans/trans-Anteil im Produkt liefert. Bei einer rein isothermen Betriebsweise eines einzelnen Hauptreaktors ohne Nachreaktor, würde man zunächst zu niedrige trans/trans-Anteile und einen hohen Anteil an nicht umgesetztem MDA erhalten. Durch die Möglichkeit den Stoffstrom gezielt adiabatisch hydrieren zu lassen, kann die Temperaturentwicklung und damit die Isomerisierung im Nachreaktor vorteilhaft gesteuert werden.

Bei einer weiter verbesserten Variante ist die leitungstechnische Verschaltung derart, dass auch der Nachreaktor beim Betrieb von mindestens einem Hauptreaktor umgangen entweder kann (Bypass 2), insb. aber beim Betrieb von den beiden in Reihe geschalteten Hauptreaktoren umgangen werden kann. Bei der Verschaltungsvariante Bypass 2, übernimmt der in Strömungsrichtung als zweiter durchflossene Hauptreaktor mind. zeitweise die Funktion des Nachreaktors, so dass die Anlage ohne bzw. weitgehend ohne Leistungsabfall und/oder Änderungen der Produktqualität, insb. des jeweiligen Anteils an Isomeren im Isomerengemisch betrieben werden kann.

Bei einer weiteren Ausführungsform der Anlage, kann es vorteilhaft sein, dass in der Leitung zwischen dem mind. einen Hauptreaktor und dem zweiten Hauptreaktor mind. ein gemeinsamer Wärmetauscher angeordnet ist. Dieser gemeinsame Wärmetauscher ist in einem zentralen Ast beider Kühlmittelumläufe angeordnet. Leitungsführung und Leitungsverschaltung ist dabei derart, dass nach dem gemeinsamen Wärmetauscher das Kühlmedium zuerst in den stromaufwärtsbefindlichen (ersten) Hauptreaktor eingeleitet wird, in dem die stärker exotherme Reaktion abläuft. Anschließend wird über eine Leitung das bereits erwärmte Kühlmedium in den stromabwärts befindlichen zweiten Hauptreaktor geleitet, so dass dieser mit einer vom ersten Hauptreaktor unterschiedlichen Vorlauftemperatur beschickt wird.

Der Vorteil besteht darin, dass es überraschenderweise beobachtet wurde, dass insb. die sehr genaue Regelung der ersten, stark exothermen Reaktionsphase für die Produktqualität entscheidend ist, so dass auf den baulichen und regelungstechnischen Aufwand eines weiteren, vollständig unabhängigen zweiten Kühlkreislaufs verzichtet werden kann. Dies insb. auch deshalb, weil über die Regelung der Vorlauftemperatur des zu den beiden Hauptreaktoren seriell nachgeschalteten Nachreaktors die vollständige Reaktion sichergestellt und geregelt werden kann, insb. der gewünschte, niedrige trans/trans-Isomerenanteil eingeregelt werden kann.

Bei einer Anlagenvariante mit noch besserer Regelbarkeit kann vorgesehen werden, dass in der jeweiligen (Kreuz-)Leitung der Kühlkreisläufe, mit denen der Kühlmittelauslass des ersten Reaktors mit dem Kühlmitteleinlass des zweiten Reaktors verbunden ist, eine bedarfsweise schaltbarer und regelbarer Wärmetauscher (Nachkühler) angeordnet ist.

Vorliegend werden im Wesentlichen folgende Energiekopplungen (EK) betrachtet, wie eine integrierte Energiekopplung oder eine direkte Energiekopplung, wobei die integrierte EK eine integrierte Energiekopplung meint, unterteilt in
a. eine integrierte stoffbasierte Energiekopplung (integrierte stoffbasierte EK) von mind. zwei Stoffströmen in einem Wärmetauscher im indirekten Wärmeaustausch meint, d.h. eine bauliche Integration in einem einzigen Wärmetauscher (Apparat), d.h. wo vormals zwei Wärmetauscher vorgesehen waren, sind beide Wärmetransportaufgaben einer baulichen Einheit (Wärmetauscher) integriert,
b. eine integrierte medienbasierte Energiekopplung (integrierte medienbasierte EK) von mind. zwei Wärmetauschmedien in einem Wärmetauscher im indirekten Wärmeaustausch meint, d.h. eine bauliche Integration in einem einzigen Wärmetauscher (Apparat);

Die vorgenannten EK können als direkte Energiekopplung (direkte EK) ausgestaltet sein, indem eine serielle EK oder serielle Verschaltung von mind. zwei Wärmetauschern vorgesehen ist.

Bei einer weiteren Ausführungsform der Anlage, kann es vorteilhaft sein, dass die Trenneinheit einen Trennkessel umfasst, der mit dem Hauptreaktor oder dem Nachreaktor über eine Leitung verbunden ist, so dass das Stoffgemisch in diesen einleitbar ist. Insb. ist der in Strömungsrichtung letzte Reaktor mit dem Trennkessel verbunden. Hierbei umfasst der Trennkessel:
- einen Kopfauslass,
- einen Boden-/Sumpfauslass und
- einen beheizbaren Sumpfumlauf mit mind. einem Wärmetauscher und/oder ist mit diesem verbunden, wobei in die (Kopf-)Leitung eine Kondensationseinheit zur mind. teilweisen Verflüssigung (Kondensation) des Lösungsmittels eingebunden ist, und wobei stromabwärts der Kondensationseinheit

- ein (Sammel-)Behälter und/oder
- eine Verbindungseinheit/-knoten mit bzw. in die (Rück-)Leitung für das Lösungsmittel angeordnet ist. Der Sumpfumlauf umfasst weiterhin eine Pumpe.

Der Sumpfumlauf des Trennkessels wird bei einer Temperatur von 130 °C bis 150 °C, idealerweise 135 bis 145 °C betrieben. Der große Vorteil des baulich und regelungstechnisch sehr einfachen Trennkessels ist, dass die Siedetemperatur des Gemisches durch die Druckabsenkung ebenfalls gesenkt wird, so dass nur auf diese erniedrigte Siedetemperatur im Trennkessel aufgeheizt werden muss. Weiterhin sind bereits durch diese Maßnahme ca. 90 % des in der Eduktmischung vorliegenden Lösungsmittels, insb. THF, abtrennbar. Ein Kopfkreislauf oder Rücklaufstrom, wie in einer Kolonne, ist hierzu nicht erforderlich. Der zur ersten Trennkolonne weitergeleitete Stoffstrom weist somit vorteilhafterweise nur eine verbleibende Lösungsmittelkonzentration von ca. 20 bis 40 Gew.% auf, idealerweise 25 bis 35 Gew.%. Somit kann die erste Trennkolonne baulich kleiner ausfallen und ist aufgrund der geringeren Masse des Stoffstroms energetisch sparsamer betreibbar.

Durch diesen Entspannungsschritt einer zwei- oder mehrstufigen Verschaltung von Trennkesseln mit direkter Wärmeintegration der ersten Trennstufe, kann der Energiebedarf dieses Entspannungsschrittes durch um 40% und mehr verringert werden.

Bei einer Variante dieser Ausführungsform der Anlage und des nachstehend beschriebenen Verfahrens, kann es vorteilhaft sein, wenn die Trenneinheit mind. einen weiteren (zweiten) Trennkessel umfasst, der stromabwärts zum ersten Trennkessel angeordnet ist, wobei der Sumpfauslass des ersten Kessels mit dem Einlass (Feed) des zweiten Trennkessels verbunden ist, wobei der zweite Trennkessel ebenfalls einen Kopfauslass, einen Boden-/Sumpfauslass und einen beheizbaren Sumpfumlauf mit mind. einem Wärmetauscher umfasst. Hierbei führt eine (KopfLeitung von dem (Kopf-)Auslass des zweiten Kessels zu der Kondensationseinheit und/oder in die (Kopf-)Leitung des ersten Kessels. Vorteilhafterweise ist in der zum Einlass des zweiten Kessels führenden Leitung eine Druckregeleinheit vorgesehen, so dass der erste Kessel bei einer ersten Temperatur und einem ersten Druck betreibbar ist und der zweite Kessel bei einer zur ersten Temperatur tieferen zweiten Temperatur und einem gegenüber dem ersten Druck niedrigeren Druck betreibbar ist. Die Druckregeleinheit ist vorteilhafterweise ein steuer- und regelbares Ventil oder Drossel. Zur Bildung und Abgrenzung der Druckniveaus in den beiden Trennkesseln kann vorteilhafterweise eine analoge Druckregeleinheit in der Kopfleitung vorgesehen werden.

Bei einer weiteren Ausführungsform der Anlage, kann es vorteilhaft sein, dass die Kondensationseinheit der ersten Trennstufe mind. zwei Wärmetauscher umfasst, wobei zwischen den beiden Wärmetauschern der Kondensationseinheit die Verbindungseinheit/-knoten für die (Kopf-)Leitung des zweiten Trennkessels mit der (Kopf-)Leitung des ersten Trennkessels angeordnet ist.

Hierbei ist vorteilhafterweise eine Druckregeleinheit in der Kopfleitung vom ersten Trennkessel zum Sammelbehälter stromaufwärts zum Leitungsknoten vorgesehen, an dem die Kopfleitung des zweiten Trennkessels in die zum Sammelbehälter führenden Kopfleitung mündet. Durch diese beiden Wärmetauscher der Kondensationseinheit in der zum Sammelbehälter führenden Leitung ist es möglich, den jeweiligen Stoffstrom auf dem jeweils vorliegenden Temperatur- und Druckniveau des zugehörigen Trennkessels zu halten.

Gemäß einer weiteren Ausführungsform der Anlage, kann nachfolgende Verschaltung der Wärmetauscher und damit die Weiterleitung des Mediums vorteilhaft sein. Eine vorteilhafte Verschaltung kann darin bestehen, wenn eine (Medien-)Leitung vom (Medien-)Ausgang der (ersten) Kondensationseinheit in der Kopfleitung des ersten Trennkessels, insb. mind. einer der Wärmetauscher, zum Eingang des (Sumpf-)Wärmetauschers des zweiten Trennkessels führt. Auf diese Weise kann insbesondere der vollständige Energiebedarf des im Sumpfkreislauf des zweiten Trennkessels eingebundenen Wärmetauschers gedeckt werden.

Bei einer vorteilhaften Verfahrensführung beträgt das Temperaturniveau in der (Kopf-)Leitung des ersten Trennkessels 110 bis 130 °C, idealerweise 115 bis 120 °C, bei einem Druck von 3 bis 5 bar, idealerweise von 3,5 bis 4,5 bar. Nach Kondensation in der Verdampfereinheit des zweiten Trennkessels ist die Temperatur des Stoffstroms in der (Kopf-)Leitung um ca. 8 bis 20 °C abgesenkt.

Die Temperatur in der (Kopf-)Leitung des zweiten Trennkessels beträgt 80 bis 100 °C, idealerweise 85 bis 95 °C, wobei der Druck im Bereich von 1,0 bis 2 bar liegt, idealerweise 1,1 bis 1,5 bar.

Bei einer alternativen Ausführungsform der Anlage hinsichtlich den zweistufigen Trennkesseln, kann es vorteilhaft sein, wenn die Trenneinheit eingangs in der ersten Trennstufe als Trennkessel mind. zwei Verdampfer umfasst und/oder im Wesentlich hieraus gebildet wird, welche in Reihe geschaltet sind, wobei in der (Kopf-)Leitung mind. eines der beiden Verdampfers als Kondensationseinheit mind. ein Wärmetauscher vorgesehen ist, der als Kondensator ausgebildet ist. Weiterhin. Gemäß einer vorteilhaften Ausführungsform kann mind. einer der Verdampfer als so genannter Kettle-Type-Verdampfer ausgebildet sein, insb. sind vorteilhafterweise beide Verdampfer als Kettle-Type-Verdampfer ausgebildet. Weiterhin ist stromabwärts des Wärmetauschers in der (Kopf-)Leitung
- ein (Sammel-)Behälter und/oder
- eine Verbindungseinheit/-knoten mit/in die (Rück-)Leitung für das Lösungsmittel angeordnet.

Die Verdampfer umfassen dabei zwei Teile. Einen Wärmetauscher-Teil (WT-Teil) und einen Kessel-Teil (K-Teil), wobei der WT-Teil in den K-Teil hineinragt oder darin vollständig angeordnet ist. Der WT-Teil wird im Wesentlichen durch mind. einen Wärmetauscher gebildet. In dem WT-Teil strömt in einem geschlossenen Leitungs- oder Kanalsystem eine erstes Fluid (Wärmetauschmedium, Stoffstrom) und in dem K-Teil, strömt eine zweites Fluid (Wärmetauschmedium, Stoffstrom), wobei im K-Teil durch den Energieeintrag des WT-Teils, bzw. dessen Wärmetauschers, eine mind. teilweise Verdampfung des im K-Teils vorliegenden Fluids erfolgt.

Die Reihenschaltung der Verdampfer erfolgt vorteilhafterweise in doppelter Hinsicht, wie folgt beschrieben:
a) vom Nachreaktor für eine (Feed-)Leitung in das K-Teil des ersten Verdampfers, der WT-Teil des ersten Verdampfers wird mit einem Heizmedium betrieben,
b) der (erste) Kopfauslass im K-Teil des ersten Verdampfers ist über eine Leitung mit dem Eingang des WT-Teils des zweiten Verdampfers verbunden,
c) der (Sumpf-)Auslass des ersten Verdampfers ist mit dem Sumpfeinlass des zweiten Verdampfers verbunden,
d) der Auslass des WT-Teils des zweiten Verdampfers ist mit der zum (Sammel-)Tank führenden Leitung verbunden, in welcher der Wärmetauscher eingebunden ist, wobei die (Kopf-)Leitung aus dem zweiten Verdampfer ebenfalls in die zum (Sammel-)Tank führende Leitung mündet, und
e) der (Sumpf-)Auslass des zweiten Verdampfers ist über eine Leitung mit der ersten Trennkolonne als (Feed-)Leitung verbunden.

Vorteilhafterweise sind zur Herstellung und Abgrenzung von unterschiedlichen Druckniveaus folgende Druckregeleinheiten vorgesehen:
- in der (Sumpf-)Leitung vom (Sumpf-)Auslass des ersten Verdampfers zum (Sumpf-)Einlass des zweiten Verdampfers und
- in der vom Auslass des WT-Teils des zweiten Verdampfers zum (Sammel-)Tank führenden Leitung, insb. stromaufwärts zur Einleitung der (Kopf-)Leitung des zweiten Verdampfers in ebendiese Leitung und stromaufwärts zu dem dortigen Wärmetauscher.

Bei einer vorteilhaften Prozessführung wird die erste Trennkolonne auf einen zum zweiten Verdampfer unterschiedlichen, tieferen Druckniveau betrieben, so dass die Anlage vorteilhafterweise in der (Sumpf-)Leitung vom (Sumpf-)Auslass des zweiten Verdampfers zum Einlass in die erste Trennkolonne ebenfalls eine Druckregeleinheit aufweist. Bei dieser Variante der Prozessführung wird die erste Trennkolonne bei einem um 3 bis 10 bar niedrigeren Druckniveau betrieben, idealerweise bei einem um 3,5 bis 5,5 bar niedrigeren Druckniveau.

Somit kann bei einer vorteilhaften Ausführungsform der Anlage vorgesehen sein, dass das WT-Teil des ersten Verdampfers mit einer Medienleitung verbunden ist, und wobei die Leitungsführung zwischen den Verdampfern derart ist, dass der PACM reiche Stoffstrom als Hochsiedeanteil im Sumpf der beiden Verdampfer geführt wird.

Hierbei wird in analoger Weise der lösungsmittelreiche Strom, der dann bspw. einem (Sammel-)Tank zugeführt und/oder zurück die Konditionierungseinheit geleitet wird aus der niedersiedenden (Dampf-)Phase gebildet und mind. teilweise auskondensiert.

Bei einer Ausführungsform der Anlage, kann es somit vorteilhaft sein, wenn eine (Stoff-)Leitung für den lösungsmittelreichen Stoffstrom vom Kopfanschluss des K-Teils des ersten Verdampfers zum Einlass des WT-Teils des zweiten Verdampfers führt, und wobei eine Leitung vom Auslass des WT-Teils zu
- einem (Sammel-)Behälter und/oder
- einer Verbindungseinheit/-knoten mit/in die (Rück-)Leitung für das Lösungsmittel führt.

Die Lösungsmittelführende (Rück-)Leitung ist mit der Konditionierungseinheit und/oder mind. einem geeigneten Sammeltank verbunden.

Von der Erfindung ist weiterhin ein Verfahren umfasst zur kontinuierlichen, katalytischen Hydrierung von Methylendianilin (MDA; Edukt1), insb. 4,4`-Diaminodiphenylmethan, mit einem Wasserstoffgeber (Edukt2), insb. einem gasförmigen Wasserstoffgeber, vorzugsweises Wasserstoff (H2),
wobei die Herstellung mittels einer industriellen Anlage erfolgt. Erfindungsgemäß ist hierbei die Anlage nach mindestens einem der vorstehenden Ausführungsformen oder Varianten ausgebildet.

Bei einer bevorzugten Ausführungsform des Verfahrens kann vorgesehen werden, dass eine kontinuierliche, katalytische Produktion von Methylenbis(cyclohexylamin) erfolgt, insb. die Produktion von 4,4'-Diaminodicyclohexylmethan (PACM), bevorzugt 4,4'-Diaminodicyclohexylmethan (PACM) mit niedrigen Anteilen an trans/trans-Isomeren. Bei einer bevorzugten Verfahrensvariante erfolgt eine kontinuierliche, katalytische Herstellung von Methylenbis(cyclohexylamin), insb. eine Produktion von PACM, nach der Formel (I)

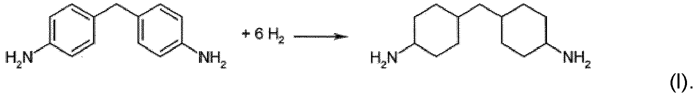

Vorteilhafterweise ist das Lösungsmittel aus der nachfolgenden Gruppe von Stoffen: Cyclohexan, Dioxan, Tetrahydrofuran (THF), Cyclohexylamin, dicyclohexylamin, Methanol, Ethanol, Isopropanol, n-Butanol, 2-Butanol, 2-Methoxy-2-methylpropan (MTBE) oder Methylcyclohexan oder eine Gemisch hieraus. Hierbei kann eine vorteilhafte Ausführungsform darin bestehen, dass das Lösungsmittel in einen Gewichtsanteil von 40 bis 50 Gew.% bezogen auf MDA in der Eduktmischung vorhanden ist.

Bei einer weiteren vorteilhaften Ausführungsform des Verfahrens kann vorgesehen sein, dass der mind. eine Hauptreaktor bei einem Druck im Bereich von 60 bar bis 120 bar betrieben wird, idealerweise im Bereich von 70 bis 110 bar. Bei einer weiteren vorteilhaften Verfahrensführung kann vorgesehen werden, dass der Druck im Hauptreaktor 70 bis 100 bar beträgt, idealerweise 80 bis 90 bar beträgt. Besonders bevorzugt ist eine Druck von ca. 85 bis 90 bar. Vorteilhafterweise wird der mind. eine Hauptreaktor bei einer Temperatur von 90 bis 140 °C, idealerweise 95 bis 135 °C betrieben. Auch wenn der mind. eine Hauptreaktor vorliegend als "isotherm" bezeichnet wird, wird dieser ideale Zustand bei industrieller Anwendung nur bedingt erreicht, so dass innerhalb des Hauptreaktors wegen unvollständigem Wärmeabtransport, sich ein Temperaturgradient von ca. 5 bis 10 °C in radiale und auch in Strömungsrichtung ausbildet.

Bei einer weiteren Ausführungsform des Verfahrens kann ein weiterer Vorteil darin bestehen, dass vom Zeitpunkt t₀, dem Verfahrensstart nach Erneuerung oder Regeneration des Katalysators bis zum Zeitpunkt t₄, dem Ende des Verfahrens, bestimmt durch die Erneuerung oder Regeneration des Katalysators, die Betriebstemperatur des Hauptreaktors erhöht und Temperatur im Stoffstrom im Einlass (Feed) des Nachreaktors gleichgehalten oder gesenkt wird, wobei die Temperaturerhöhung oder -Absenkung linear und/oder schrittweise erfolgt.

Bei einer weiteren vorteilhaften Ausführungsform des Verfahrens kann vorgesehen sein, dass der Druck im Hauptreaktor 70 bis 100 bar beträgt, idealerweise 80 bis 90 bar.

Bei einer weiteren vorteilhaften Ausführungsform des Verfahrens kann vorgesehen sein, dass - die Temperatur am Eingang des Hauptreaktors im Wesentlichen der Temperatur am Eingang des Nachreaktors entspricht, wobei im Wesentlichen einen Bereich oder Unterschied von +/- 10 °C meint und/oder der Druck am Eingang des Hauptreaktors im Wesentlichen dem Druck am Eingang des Nachreaktors entspricht, wobei im Wesentlichen einen Bereich oder Unterschied von +/- 5 bar meint.

Bei einer weiteren vorteilhaften Ausführungsform des Verfahrens kann vorgesehen sein, dass das MDA (Edukt1) eine Mischung der mind. zwei Isomere umfasst: 4,4' MDA, 2,4' MDA und 2,2' MDA.

Idealerweise ist der hohe Anteil an trans/trans PACM im Produkt im Bereich von 15 bis 30 Gew.%, idealerweise 16 bis 25 Gew.%.

Bei einer weiteren Ausführungsform des Verfahrens kann ein weiterer Vorteil darin bestehen, dass das MDA (Edukt1) eine Mischung der folgenden Monomere umfasst: 4,4'-MDA, 2,4'-MDA und 2,2'-MDA, wobei der Anteil an 4,4'-MDA vorteilhafterweise im Bereich von 75 bis 98 mol-% beträgt, idealerweise 85 bis 95 mol-%, bevorzugt 90 mol-%. Der Anteil an 2,4' MDA im Eduktgemisch beträgt vorteilhafterweise 7 bis 15 mol-%, bevorzugt 8 bis 12 mol-%, idealerweise 9 bis 10 mol-%.

Bei einer weiteren vorteilhaften Ausführungsform des Verfahrens kann vorgesehen sein, dass die erste Trennstufe der Trenneinheit zum (ersten) Trennkessel mind. einen weiteren (zweiten) Trennkessel umfasst, der stromabwärts und seriell verschaltet zum ersten Trennkessel angeordnet ist. Hierbei ist ein Sumpfauslass des ersten Trennkessels mit einem Einlass des zweiten Trennkessels verbunden, wobei der erste Trennkessel bei einer ersten Temperatur und einem ersten Druck betreiben wird und der zweite Trennkessel bei einer zur ersten Temperatur tieferen zweiten Temperatur (um 20 °C bis 40 °C geringer) und einem gegenüber dem ersten Druck niedrigeren Druck (um 1,5 bar bis 4,5 bar geringer) betrieben wird. Es ist insb. vorteilhaft, wenn eine energetische Verschaltung derart vorgenommen wird, dass der Stoffstrom der Kopfleitung (Dampfleitung) des ersten Trennkessel als Wärmequelle für den Sumpfumlauf des zweiten Trennkessels dient. Diese energetische Verschaltung kann idealerweise in einem einzigen Wärmetauscher oder in zwei über mind. eine Medienleitung gekoppelte Wärmetauscher erfolgen.

Insgesamt sollen alle Aspekte, Vorteile und Ausführungen zur Anlagen oder in Zusammenhang mit deren Beschreibung genannt sind, identisch oder in analoger Weise auch für das Verfahren gelten und umgekehrt, sofern nicht etwas Abweichendes dargelegt ist und/oder eine technische Unmöglichkeit der analogen Anwendung gegeben ist.

Nachfolgend wird die erfindungsgemäße Lösung anhand von Ausführungsbeispielen im Detail beschrieben. Es zeigen:
- Fig. 1: eine Anlage als Prozessfließbild
- Fig. 2: eine erste Ausführungsform einer zweistufigen Entspannung
- Fig. 3: eine weiter Ausführungsform einer zweistufigen Entspannung
- Fig. 4: eine Variante der Ausführungsform nach Figur 3,
- Fig. 5: eine weitere Ausführungsform der Reaktionseinheit und
- Fig. 6: eine weiter Ausführungsform der Reaktionseinheit.

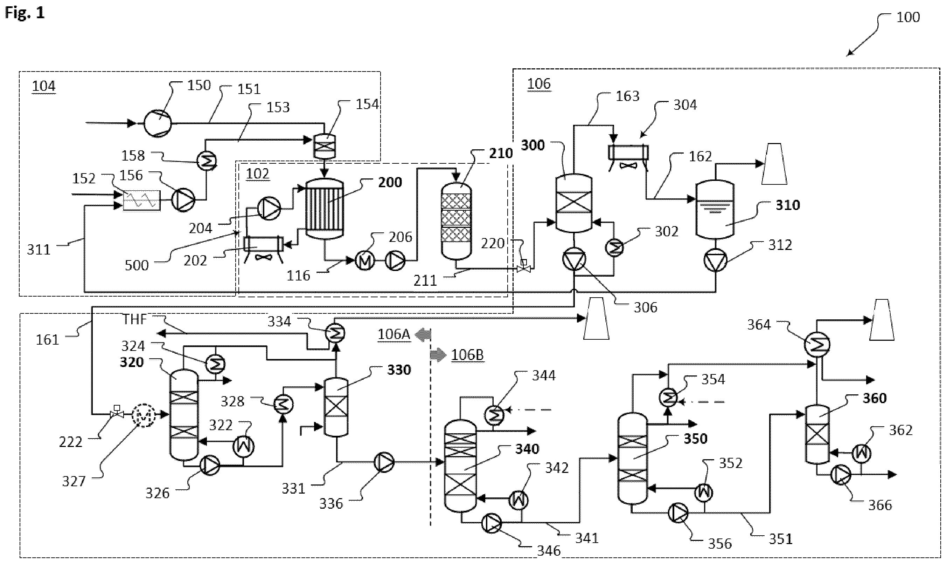

Die Figur 1 zeigt die Anlage 100 zur kontinuierlichen Herstellung von 4,4'-Diaminodicyclohexylmethan (PACM) durch katalytische Hydrierung von Methylendianilin (MDA; Edukt1) mit einem Wasserstoffgeber (Edukt2), wobei der Wasserstoffgeber als gasförmiger Wasserstoff (H2) zugeführt wird. Die Anlage 100 umfasst eine Konditionierungseinheit 104 für die Edukte, eine Reaktoreinheit 102 und eine Trenneinheit 106.

Die Konditionierungseinheit 104 ist gestrichelt umrahmt und umfasst (Zu-)Leitungen für das Edukt1 und den Wasserstoff (Edukt2) sowie das Lösungsmittel. Weiterhin umfasst die Konditionierungseinheit eine Verdichtereinheit 150, nachfolgend Verdichter genannt in der den Wasserstoff zuleitenden Leitung 151, einen Mischer 152 in der das Lösungsmittel und das Edukt1 zuführenden Leitung. Zudem sind in der die Mischung aus Edukt1 und Lösungsmittel zuführenden Leitung 153 eine Pumpe 156 und ein Wärmetauscher 158 angeordnet. Die Leitungen 151, 152 münden in einen Mischbehälter 154, der stromaufwärts zum Hauptreaktor 200 angeordnet ist. Der Mischbehälter 154 dient der intensiven Vermischung der Edukte und dessen Auslauf bildet den Zulauf für den Hauptreaktor 200.

Die Reaktoreinheit 102 ist gestrichelt umrahmt und umfasst im Wesentlichen den Hauptreaktor 200, einen Kühlumlauf 500, eine Nachreaktor 210 und einen Wärmetauscher 206 in der Zulaufleitung zum Nachreaktor 210. In den Kühlumlauf 500 ist eine Pumpe 204 und ein Wärmetauscher 202 eingebunden, wobei das umlaufende Kühlmittel im Hauptreaktor 200 die mit Katalysatormaterial befüllten Trägerelemente umströmt. Im gezeigten Beispiel werden die mit Katalysatormaterial befüllten Trägerelemente im Gleichstromrichtung angeströmt. Der Nachreaktor 210 ist über die Leitung 211 mit dem Trenneinheit 106, bzw. deren ersten Trennstufe verbunden.

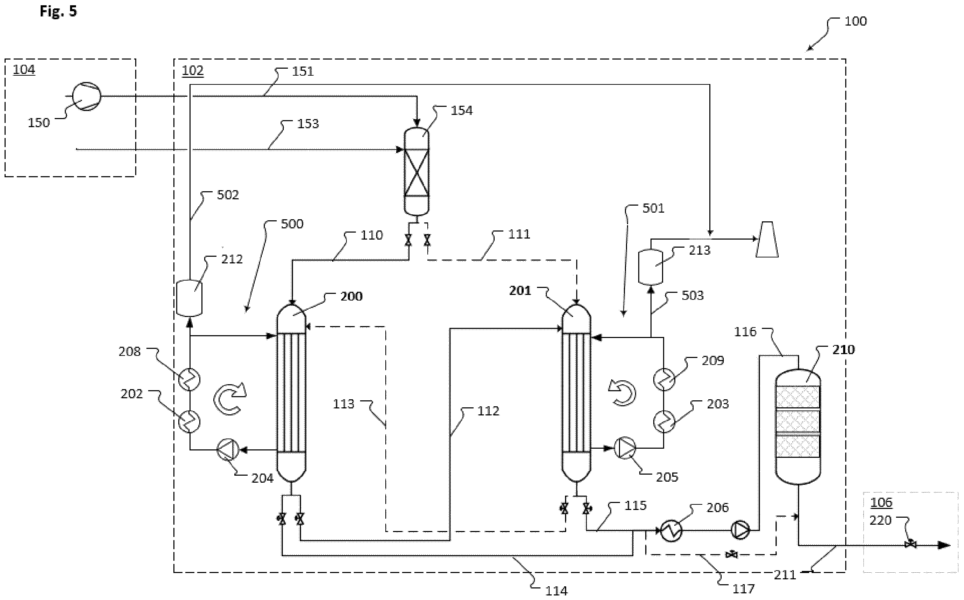

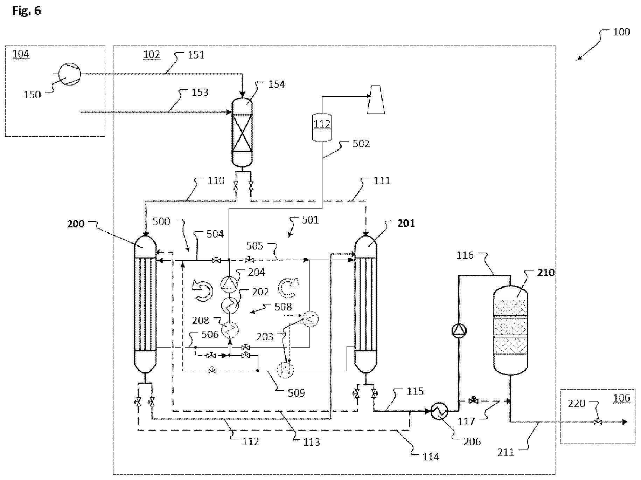

Der Wärmetauscher 202 im Kühlumlauf 500 ist als luftgekühlter Wärmetauscher 202 dargestellt, kann aber auch alternativ ausgebildet sein, um bspw. mittels eines fließenden Kühlmediums, wie bspw. ein Öl, Wasser oder eine Sole, um im indirekten Wärmeaustausch das Kühlmedium des Kühlumlaufs 500 zu temperieren. In den Kühlumlauf 500 ist weiterhin bei einer nichtdargestellten Anlagenvariante ein Wärmetauscher eingebunden, analog den Wärmetauschern 208, 209 der Figuren 5, 6. Dieser wird mit einem Heizmedium betrieben und dient im Anfahrschritt des Hauptreaktors 200 zur Temperierung des Hauptreaktors 200 auf ca. 80 bis 100 °C, idealerweise auf eine Temperatur von 85 °C bis 95 °C. In dem gezeigten Anlagen- und Verfahrensbeispiel, bei dem Ziel ein möglichst niedriges trans/trans-Isomerenverhältnis von ca. 17 bis 23 Gew.% zur realisieren, wird der mit frischem oder regeneriertem Katalysator befüllte Hauptreaktor 200 auf eine Temperatur von ca. 90 °C mittels des Wärmetauschers 208 vorgeheizt. Der Hauptreaktor 200 wird bei einem Druck von 87 bis 88 bar betrieben.

Die Trenneinheit 106 ist gestrichelt umrahmt und umfasst eine Mehrzahl von Trennapparaten zur Abtrennung des Lösungsmittel insb. in einer ersten Trennstufe und des Produkts PACM, insb. in einer zweiten Trennstufe 106B, von restlichem Edukt und Nebenprodukten. Die erste Trennstufe (nicht ausgewiesen) umfasst einen Trennkessel 300 (Flashbehälter) an den eine Sumpfumlauf angeschlossen ist, in den ein Wärmetauscher 302 und eine Pumpe 306 eingebunden ist. Der Kopfauslass des Trennkessels 300 ist mit einem Wärmetauscher 304, einem Kondensator, verbunden, zu dem stromabwärts ein Sammelbehälter 310 für das Lösungsmittel vorgesehen ist. Mittels des Kondensators 304 werden ca. 80% des Lösungsmittels, vorliegend THF, auskondensiert und könnten gesammelt bzw. zurückgeleitet werden. In der Flashstufe ist ein Energiebedarf für den Wärmetauscher 302 im Sumpfumlauf des Trennkessels 300 von ca. 1400 kW erforderlich.

Die Kondensationseinheit 304 ist als Wärmetauscher in der Bauform eines luftgekühlten Apparates dargestellt, kann aber auch alternativ ausgebildet sein, um bspw. mittels eines fließenden Kühlmediums, wie bspw. einer Kühlwasser oder ein zur Wärmeintegration geeignetes Medium, im indirekten Wärmeaustausch den eingangs dampfförmigen Stoffstrom mind. teilweise zu kondensieren und zu kühlen.

Die hierin genannten Energiemengen sind kalkuliert für eine Anlagenleistung des Produktes PACM bei der genannten Synthesereaktion von ca. 3,37 t/h, sowie eine Nebenproduktleistung von ca. 0,4 t/h an HB und ca. 0,05 t/h an LB. Das Verhältnis der Massenströme von THF zu MDA betrugt 4,2 bis 4,5.

Weiterhin umfasst die erste Trennstufe der Trenneinheit 106 zur weitergehenden Abtrennung des Lösungsmittels einen erste Trennkolonne 320 und eine zweite Trennkolonne 330, wobei die zweite Trennkolonne 330 als Stripping Kolonne ausgebildet ist. Hierzu wird vorteilhafterweise Stickstoff (N2) als Stripping-Medium eingesetzt, das im Gegenstrom zum Stoffstrom durch die Kolonne geleitet wird. Mittels der im Sumpfauslass des Trennkessels 300 befindlichen Pumpe 306 ist der an Lösungsmittel verarmte Stoffstrom über die Leitung 161 zur ersten Trennkolonne 320 ableitbar. In der Leitung 161 ist eine Entspannungseinheit 222 vorgesehen, die im gezeigten Beispiel als regelbares Ventil ausgebildet ist. Der ersten Trennkolonne 320 wird der Stoffstrom über einen zentralen Zulauf wie dargestellt eingeleitet. Weiterhin ist stromaufwärts zur ersten Trennkolonne 320 ein (Vorlauf-)Wärmetauscher 327 (gestrichelt dargestellt) angeordnet, der eine Option zur Beheizung der ersten Trennkolonne 320 darstellt.

In der ersten Trennkolonne 320 wird die Lösungsmittelkonzentration von 30 Gew.% im Zulauf über den produktreichen Stoffstrom der Leitung 161 auf ca. 2 Gew.% an restlichem Lösungsmittel verringert.

Die erste, mit (Struktur-)Packungen bestückte, Trennkolonne 320 ist an einen Sumpfumlauf angeschlossen, in den ein Wärmetauscher 322 und eine Pumpe 326 eingebunden sind. Weiterhin ist am Kolonnenkopf der ersten Trennkolonne 320 ein Kopfumlauf angeordnet, in den ein Wärmetauscher 324 eingebunden ist, der als Kondensator ausgebildet ist. Aus dem Kopfumlauf führen zwei Ableitungen für den lösungsmittelreichen Stoffstrom, wobei eine der beiden Ableitungen in die Kopfableitung der stromabwärts befindlichen Trennkolonne 330 (Stripping-Kolonne) einmündet.

Insbesondere ist das in der ersten Trennstufe abgetrennte Lösungsmittel über die Leitung 311 in einen Sammeltank (nicht dargestellt) und/oder den Mischer 152 der Konditionierungseinheit 104 leitbar. In der vom Nachreaktor 210 zum Trennkessel 300 führenden Leitung 211 ist eine Druckregeleinheit 220 vorgehen, die im vorliegenden Beispiel als steuerbares Ventil ausgebildet ist.

Über die Leitung 321 wird aus dem Sumpfauslass der ersten Trennkolonne 320 der produktreiche Stoffstrom mit einem Restanteil von ca. 2 Gew.% Lösungsmittel (THF) in den Kopf der zweiten Kolonne 330, der Stripping-Kolonne, geleitet. In dieser Leitung 321 ist ein Wärmetauscher 328 eingebunden. Die zweite Kolonne 330 weist mind. eine innere Packung auf, wobei unterhalb der Packung eine Gaszuleitung für insb. ein Inertgas (Strippinggas) angeordnet ist, so dass der eingeleitete produktreiche Stoffstrom im Gegenstromprinzip zum Strippinggas die zweite Kolonne 330 durchströmt und von Lösungsmittel weiter abgereichert wird. In dem gezeigten Anlagenbeispiel wird Stickstoff (N2) als Strippinggas eingesetzt. Der lösungsmittelreiche Dampf wird über den Kopfauslass in einen Kondensator 334 geleitet und gemeinsam mit dem von Kopfauslass der ersten Trennkolonne 320 kommenden und die Kopfableitung der zweiten Trennkolonne 330 eingeleitete Dampfstrom dem Kondensator 334 zugeleitet. Der im Wärmetauscher 334 auskondensierte Lösungsmittelstrom wird zur Konditionierungseinheit 104 zurückgeleitet, wobei der nicht-kondensierbare Anteil aus dem Wärmetauscher 334 abgeleitet und bspw. vollständig thermisch oxidiert wird.

Vom Sumpfauslass der zweiten Trennkolonne 330 wird der produktreiche Stoffstrom über die Leitung 331, in die die Pumpe 336 eingebunden ist, der dritten Trennkolonne 340 zugeführt. Die zweite Trennstufe 106B der Trenneinheit 106 dient insb. der Abtrennung der Nebenprodukte LB und/oder HB vom Produkt PACM.

Die zweite Trennstufe umfasst im Wesentlichen drei Trennkolonnen, wobei der dritten Trennkolonne 340, die erste der zweiten Trennstufe, der Stoffstrom zentral zugeführt wird. Die dritte Trennkolonne ist mit einem Sumpfumlauf verbunden, in den ein Wärmetauscher 342 und eine Pumpe 346 eingebunden sind. Der produktreiche Stoffstrom wird über die Leitung 341 aus dem Sumpfablauf zur vierten Trennkolonne 350 geleitet. Weiterhin ist die dritte Trennkolonne 340 mit einem Kopfumlauf verbunden, in den ein Wärmetauscher 344 eingebunden ist. Aus diesem Kopfumlaufwird auskondensiertes LB als erstes Nebenprodukt abgeleitet.

Der produktreiche Stoffstrom wird über die Leitung 341 der vierten Trennkolonne 350 zentral zugeführt. Die vierte Trennkolonne 350 ist mit einem Sumpfumlauf verbunden, in den ein Wärmetauscher 352 und eine Pumpe 356 eingebunden sind. Weiterhin ist die vierte Trennkolonne 350 mit einem Kopfumlauf verbunden, in den ein als Kondensator ausgebildeter Wärmetauscher 354 eingebunden ist. Der produktreiche Stoffstrom wird über die Kopfableitung aus dem Kopfumlauf als Kondensat ausgeleitet. Vorliegend wird gemäß dem gezeigten Beispiel, der nicht-kondensierte Dampf- und/oder Gasstrom in die Kopfausleitung der stromabwärtsbefindlichen fünften Trennkolonne 360 eingeleitet. Nachfolgend wird über einen weiteren Wärmetauscher 364 (Kondensator) Produkt weiter auskondensiert und ausgeleitet. Der produktarme Stoffstrom wird über die Sumpfableitung der vierten Trennkolonne 350 über die Leitung 351 abgeleitet und der fünften Trennkolonne 360 in deren Kopfteil eingeleitet. Dieser Stoffstrom ist stark angereichert an HB, einem zweiten Nebenprodukt. Die fünfte Trennkolonne 360 ist mit einem Sumpfumlauf verbunden, in den ein Wärmetauscher 362 und eine Pumpe 366 eingebunden sind. Weiterhin weist die Trennkolonne 360 einen Kopfauslass auf, der zu dem vorgenannten als Kondensator ausgebildeten Wärmetauscher 364 führt. In diesem Kondensator 364 wird ein weiterer produktreicher Stoffstrom als Kondensat auskondensiert und ausgeleitet, wobei der nicht-kondensierbare Anteil gasförmig ausgeleitet wird. Dieser kann nachfolgend insb. vollständig oxidiert werden.

Die Reaktoreinheit 102 wird bzgl. des Stoffstroms auf einem ersten, hohen Druckniveau von ca. 60 bis 120 bar betrieben, die erste Trennstufe der Trenneinheit 106 wird bei einem zweiten, niedrigen Druckniveau von 4 bis 12 bar betrieben und die erste Trennkolonne 320 und die zweite Trennkolonne 330 werden auf einen dritten, geringfügig erhöhten Druckniveau von 1,05 bis 2,5 bar betrieben. In dem gezeigten Beispiel ist das erste Druckniveau 80 bis 90 bar, das zweite Druckniveau 4,5 bis 7,5 bar und das dritte Druckniveau 1,1 bis 1,2 bar.

Weiterhin wird die Reaktoreinheit 102 bzgl. des Stoffstroms auf einem ersten, hohen Temperaturniveau von ca. 90 °C bis 140°C betrieben, wobei der die erste Trennstufe der Trenneinheit 106 wird bei einem zweiten, niedrigen Druckniveau von 4 bis 12 bar betrieben und die erste Trennkolonne 320 und die zweite Trennkolonne 330 werden auf einen dritten, geringfügig erhöhten Druckniveau von 1,05 bis 2,5 bar betrieben. In dem gezeigten Beispiel ist das erste Druckniveau 80 bis 90 bar, das zweite Druckniveau 4,5 bis 7,5 bar und das dritte Druckniveau 1,1 bis 1,2 bar.

Mit der direkten Temperaturregelung des Hauptreaktors 200 über den Kühlkreislauf 500 in Reihe mit dem ungekühlten Nachreaktor 210, hat sich überraschenderweise ein gegenüber dem Stand der Technik stark verringerter Energiebedarf und eine vereinfachte, stabilere Verfahrensführung gezeigt. Ohne auf eine spezielle Interpretation gebunden zu sein, wird dieser Erfolg darin gesehen, dass der Kühlkreislauf 500 nur gezielt für die sehr heftige Anfangsreaktion zur Ableitung der exothermen Reaktionswärme ausgelegt werden muss, wobei die noch bedeutsame Nachreaktion alleinig über die Zulauftemperatur mittels des stromaufwärts befindlichen Wärmetauscher 206 eingestellt werden muss. Der Stoffstrom wird durch die bereits stark abgeschwächte Reaktion im Nachreaktor 210 nur noch um ca. 5 bis 15 °C erwärmt und kann in diesem Niveau unproblematisch in den Trennkessel entlassen werden.

Insgesamt sind in den Figuren in den Apparaten, wie Reaktoren, Trennkolonnen, Behältern etc., über die entsprechenden Symbole Einbauten, wie Packungen, Trennebenen, Trägerelemente etc. skizziert. Diese deuten jeweils vorteilhafte Ausführungsformen an, betreffend die Anzahl, Art und/oder relative Lage zur jeweils zu- oder abführenden Leitung. So ist bspw. mit der Darstellung ersten Trennkolonne 320 angedeutet, dass vorteilhafterweise der (Feed-)Stoffstrom über die Leitung 161 derart eingeleitet wird, dass zwischen dem Kopfumlauf und dem Sumpfumlauf jeweils mind. eine (theoretische) Trennebene vorhanden ist. Die Ermittlung der konkreten Art und/oder Anzahl von Trennebenen ist dem Fachmann bekannt und kann in geeigneter Weise variiert bzw. vorgesehen werden.

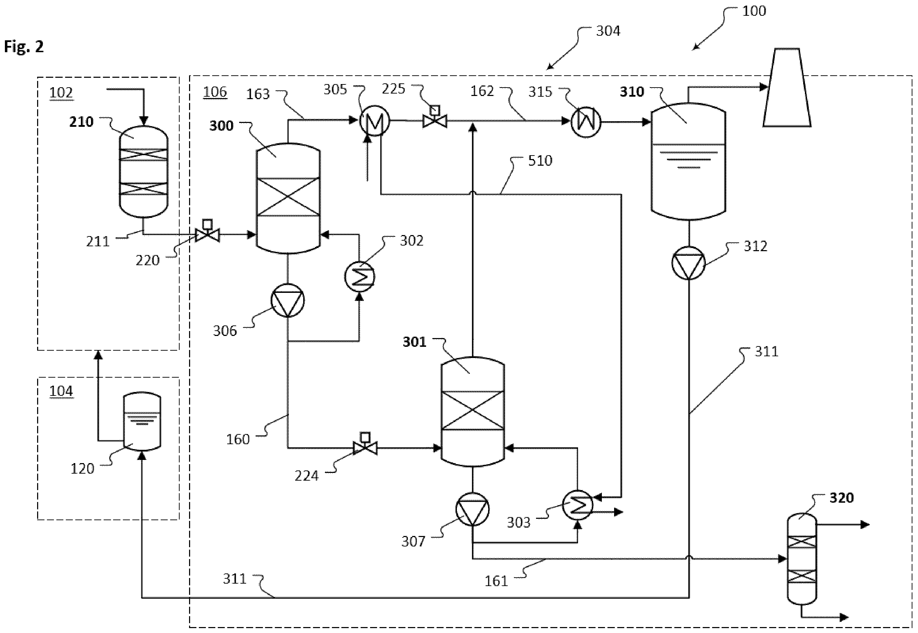

Figur 2 zeigt eine erste Ausführungsform einer zweistufigen Entspannung mit vorteilhafter Verschaltung der Wärmetauscher in der ersten Trennstufe der Trenneinheit 106. Im Unterschied zu der in Figur 1 gezeigten Variante ist stromabwärts zum (ersten) Trennkessel 300 ein weiterer (zweiter) Trennkessel 301 angeordnet. Der weitere Trennkessel 301 weist ebenfalls einen Sumpfumlauf auf, in den eine Wärmetauscher 303 und eine Pumpe 307 eingebunden sind. Der Sumpfablauf des ersten Trennkessels 300 ist mit dem Sumpfeinlauf des zweiten Trennkessels 301 über die Leitung 160 verbunden und der Sumpfauslauf des zweiten Trennkessels 301 ist über die Leitung 161 mit der ersten Trennkolonne 320 in analoger Weise zur Figur 1 verbunden. Die beiden Trennkessel 300, 301 werden auf geringfügig unterschiedlichen Druckniveaus betrieben, wozu in der verbindenden Leitung 160 zwischen den beiden Trennkessel 300, 301 eine Druckregeleinheit 224 angeordnet ist, die im vorliegenden Beispiel als steuer- und regelbares Ventil ausgebildet ist. Der produktreiche Stoffstrom wird über die Sumpfleitungen 211, 160, 161 über die beiden Trennkessel 300, 301 der ersten Trennkolonne 320 zugeführt. Der lösungsmittelreiche Dampf- oder Kondensatstrom wird über die jeweiligen Kopfauslässe der beiden Trennkessel 300, 301 in die Leitung 162 eingespeist und in den Sammelbehälter 310 eingeleitet, wobei in die Leitung 162 zwei Wärmetauscher 305, 315 eingebunden sind, die als Kondensatoren ausgebildet sind. Der erste Abschnitt der Leitung 162 vom Kopfauslass des Trennkessels 300 bis zum Wärmetauscher 304 bzw. 305, ist mit der Bezugsziffer 163 ausgewiesen.

In der Figur 2 sind die Wärmetauscher 305, 303 als zwei getrennte Apparate dargestellt, wobei im Wärmetauscher 305 (Kondensator), das Lösungsmittel (THF) bei hohem Druck kondensiert wird und im Wärmetauscher 303, wird die in den Wärmetauscherrohren freiwerdende Kondensationsenthalpie benutzt, um bei kleinerem Druck im Trennkessel 301 weiteres Lösungsmittel zu verdampfen. Die EK der beiden Wärmetauscher 305, 303 kann in einer in Figur 2 nicht gezeigten Ausführungsvariant auch als integrierte medienbasierte EK ein einem einzigen Apparat realisiert sein.

Durch die Druckregeleinheit 224 in der verbindenden (Sumpf-)Leitung 160 wird das zweite Druckniveau von 4,5 bis 7,5 bar gesplittet, indem der erste Trennkessel 300 bzw. in der Leitung 160 stromaufwärts zur Druckregeleinheit 224 gegenüber dem zweiten Trennkessel 301 bzw. dem Druckniveau in der Leitung 161 stromaufwärts zur Druckregeleinheit 222 bei einem um ca. 1,5 bis 2,5 bar erhöhten Druck betrieben wird. So ist im vorliegenden Fall das Druckniveau in der Leitung 160 auf dem Niveau von 3,5 bis 5,5 bar, insb. ca. 4,3 bar, wobei der zweite Trennkessel 301 bzw. die Leitung 161 stromaufwärts zur Druckregeleinheit 222 auf einem Druckniveau von ca. 1,0 bis 2,0 bar betrieben wird, insb. ca. 1,3 bar. Weiterhin ist eine Druckregeleinheit 225 in der (Dampf-)Leitung 162 stromaufwärts zur Einleitung kommend von der Kopfausleitung des zweiten Trennkessels 301 vorgesehen, so dass auch in den Dampfleitung 162, 163 in analoger Weise zwei Druck- und Temperaturniveaus einstellbar sind. In dem gezeigten Beispiel verlässt der Dampf den ersten Trennkessel 300 bei ca. 4,0 bis 4,5 bar und einer Temperatur von ca. 110 bis 120 °C und der Dampf des zweiten Trennkessels 301 wird mit ca. 1,1 bis 1,5 bar und einer Temperatur von ca. 85 bis 95 °C entlassen und in die Leitung 162 eingeleitet.

Betreffend den Medienfluss und den Energiebedarf, weist der Stoffzulauf über die Leitung 211 einen Temperatur von ca. 115 bis 130 °C auf und der Sumpfumlauf wird bei einer hierzu um ca. 10 °C erhöhten Temperatur betrieben, wobei der Wärmetauscher im Sumpfumlauf des ersten Trennkessels 300 mit Dampf als (Wärmetausch-)Medium betrieben wird. Das dem ersten Kondensator 305 zugeleitete (Kühl-)Medium, wie bspw. Wasser, wird erwärmt und als (Heiz-)Medium über die Leitung 510 dem im Sumpfkreislauf des zweiten Trennkessels 301 eingebundenen Wärmetauscher 303 zugeleitet. Hierdurch konnte im Vergleich zur einstufigen Flasheinheit und Kondensation mittels des einen Kondensators 304, wie in der Figur 1 gezeigt, der Energiebedarf für den Wärmetausch im Sumpfumlauf der Trennkessel 300, 301 um ca. 50 % gesenkt werden, von vorher ca. 1440 kW für den Wärmetauscher 302 auf verbleibende ca. 700 kW für den Wärmetauscher 302 des ersten Trennkessels 300. Weiterhin konnte das Volumen des ersten Trennkessels 300 um ca. 20 % kleiner ausgelegt werden und die elektrische Pumpenleitung der Sumpfpumpe 302 um ca. 15 % verringert werden.

Der Sammelbehälter 310 weist neben dem Sumpfauslass einen Kopfauslass auf, über den der nicht-kondensierte Gasanteil abgeleitet werden kann, insb. zu einer vollständigen thermischoxidativen Behandlungseinheit abgeleitet werden kann.

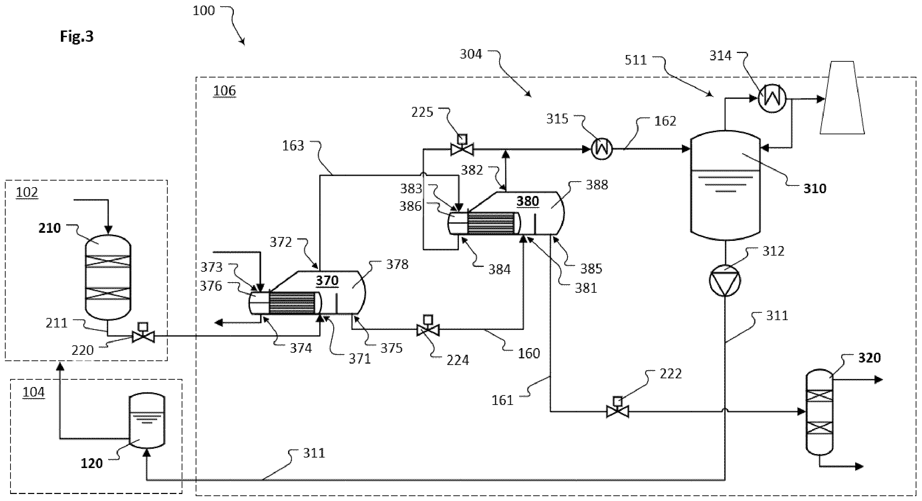

In der Figur 3 ist eine weitere Ausführungsform eine zweistufigen Entspannung mit einer besonders vorteilhaften Verschaltung in der ersten Trennstufe der Trenneinheit 106 gezeigt, wobei die EK als eine serielle EK realisiert ist. Hierbei sind statt der Trennkessel zwei Verdampfer 370, 380 in Reihe angeordnet bzw. kreuzweise verschaltet, wie nachstehend ausgeführt. Die Verdampfer 370, 380 sind so genannte Kettle-Type-Verdampfer, die einen für das strömende Fluid geschlossenen Wärmetauscher-Teil 376, 386 (WT-Teil) und einen für das strömende Fluid offenen Kessel-Teil 378, 388 (K-Teil) aufweisen. Die WT-Teile 376, 386 weisen jeweils einen Einlass 373, 383 und einen Auslass 374, 384 auf, wobei das Fluid in geschlossenen Kanälen oder Rohren geführt wird, wie bspw. mind. einem Rohrbündel. Die K-Teile 378, 388 weisen jeweils mind. einen (Sumpf-)Einlass 371, 381, jeweils einen (Sumpf-)Auslass 375, 385 und jeweils mind. einen (Kopf-)Auslass 372, 382 auf, wobei das über insb. den mind. einen (Sumpf-)Einlass eingeleitete Fluid mittels des jeweiligen WT-Teils bzw. des zugehörigen Wärmetauschers erwärmt und mind. teilweise verdampft wird. Das jeweilige Kessel-Teil kann zweiteilig sein, wie im gezeigten Beispiel. Hierbei ist der (Sumpf-)Einlass in einem zentralen K-Teil angeordnet, in welchen auch der Wärmetauscher des WT-Teils hineinragt und der Energieeintrag in den K-Teil erfolgt. Der (Sumpf-)Auslass ist in einem seitlichen oder äußeren K-Teil angeordnet, in dem vorteilhafterweise, aber nicht zwingend, durch Einbauten eine für das flüssige Stoffgemisch beruhigte Zone gebildet ist und/oder in den der Wärmetauscher des WT-Teil nicht hineinragt.

Im gezeigten Beispiel wird der WT-Teil des ersten Verdampfers 370 mit Dampf bei einer Temperatur von ca. 120 bis 140 °C bei einem Druck von ca. 3 bar über den Einlass 373 beschickt. Der Stoffstrom von dem Nachreaktor 310 tritt über den (Sumpf-)Einlass 371 in das K-Teil 378 ein, wird dort teilweise verdampft. Der lösungsmittelreiche Dampfanteil wird aus dem (Kopf-)Auslass 372 über die Leitung 163 als Heizmedium dem WT-Teil 386 in den Einlass 383 des zweiten Verdampfers 380 geleitet und vom Auslass 384 über den Kondensator 315, die dortige Kondensation, und die Leitung 162 in den Sammeltank 310 eingeleitet. Verglichen mit der Ausführung gem. der Figur 2, sind in der gezeigten Kaskade aus den beiden Verdampfern 370, 380 der Kondensator 305 und der (Sumpf-)Wärmetauscher 303 in einem Apparat bzw. in einem Wärmetauscher, nämlich dem WT-Teil 386 des Verdampfers 380 zusammengefasst. Der Dampfanteil des zweiten Verdampfers 380 wird in analoger Weise zum Ausführungsbeispiel der Figur 2 in die Leitung 162, stromaufwärts zum Kondensator 315 eingeleitet. Der (Sumpf-)Auslass 385 ist über die Leitung 161 mit der ersten Trennkolonne 320 verbunden. Ebenfalls in analoger Weise werden die beiden Verdampfer 370, 380 auf unterschiedlichen Druck- und Temperaturniveaus betrieben, die im Wesentlichen denen der Varianten nach Fig. 2 entsprechen, so dass in der Sumpfleitung 160 vom Auslass 375 des ersten Verdampfers 370 zum Einlass 381 des zweiten Verdampfers 380, eine Druckregeleinheit 224 angeordnet ist.

Der Vorteil dieser Ausführungsform besteht in einer kompakteren Bauweise mit geringerem Flächen- und Bauraumbedarf. Weiterhin treten durch die theoretische Integration der beiden Wärmetauscher 305, 303 in den einen, innenliegenden W-Teil 386 des zweiten Verdampfers 380 weniger Wärmeverluste durch die geringeren Leitungslängen auf, hier insb. der Wegfall der Leitung 510. Aus demselben Grund verringert sich der Aufwand zur Isolation von Leitungen/-wegen.

In der gezeigten Ausführungsform der Figur 3 ist der Sammelbehälter mit einem Kopfkreislauf 511 verbunden, in den ein Wärmetauscher 314 eingebunden ist, der als Kondensator ausgebildet ist. Hierüber kann weiteres Lösungsmittel (THF) abgetrennt werden.

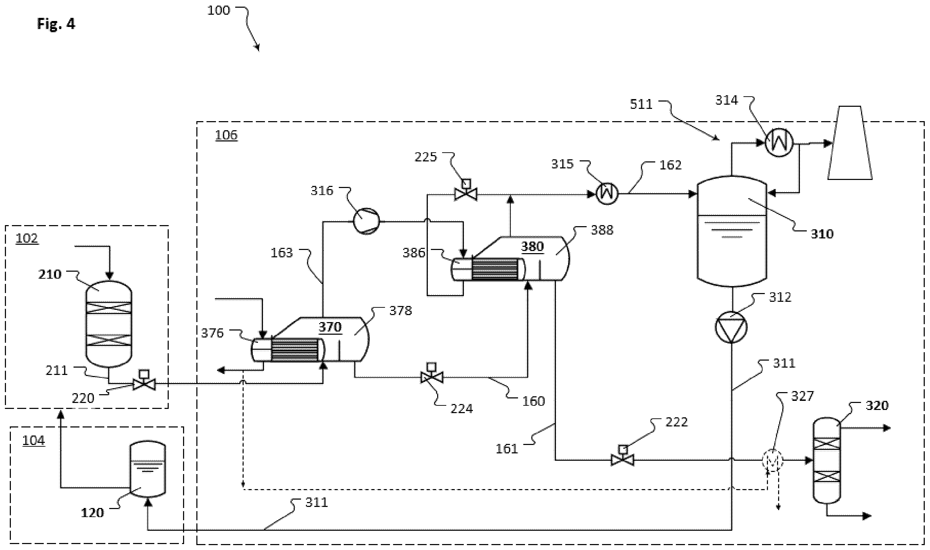

Bei einer weiter verbesserten Ausführungsform, wie sie in der Figur 4 gezeigt ist, wir ein Teil des Energiebedarfes durch elektrische Energie eingebracht, indem in der Leitung 163 ein Verdichter 390 vorgesehen ist. Hierdurch wird die Temperatur das Energieniveau im Stoffstrom angehoben und ggf. ein Teil verflüssigt, so dass der Wirkungsgrad im WT-Teil 386 erhöht ist.

Neben dem Vorteil des erhöhten Wirkungsgrades besteht ein weiterer Vorteil darin, dass der Volumenstrom in der Leitung 163 durch die Teilverflüssigung stark verkleinert wird, so dass die nachfolgenden Bauteile, insb. des WT-Teils 386 kleiner dimensioniert oder im selben Bauraum mehr Wärmeaustauschfläche im Inneren des K-Teils 388 zur Verfügung gestellt werden kann, so dass der Verdampfer 380 insgesamt einen höheren Wirkungsgrad aufweist.

Figur 4 zeigt eine weiter optionale Ausführungsform, die ebenfalls bei der Figur 3 vorgesehen werden kann. Ist stromaufwärts zur ersten Trennkolonne 320 ein (Vorlauf-)Wärmetauscher 327 vorgesehen. Vorteilhafterweise wird der abfließende Medienstrom des WT-Teils 376 des ersten Verdampfers 370, der eine Temperatur von ca. 105 bis 125 °C aufweist, zur EK als Heizmedium diesem (Vorlauf-)Wärmetauscher 327 zuführt. Auf diese Weise wird der Energiebedarf des Wärmetauschers 322 im Sumpfumlauf der ersten Trennkolonne 320 nahezu linear gesenkt. Bei einer nicht dargestellten Ausführungsform ist eine integrierte stoffbasierte EK vorgesehen, indem die Wärmetauscher 376 und 327 als baulich ein (einziger) Apparat integriert ausgeführt sind.

Figur 5 zeigt eine verbesserte Variante der Reaktoreinheit 102. Hierbei sind zwei Hauptreaktoren 200, 201 schaltbar zueinander in Reihe geschaltet. Die steuer- und/oder regelbaren Ventile/- einheiten sind zum Teil in der Figur 5 eingezeichnet, weitere können vom Fachmann bedarfsweise vorgesehen werden, um den sicheren Betrieb der beiden Hauptreaktoren 200, 201 sicherzustellen. Die beiden Hauptreaktoren 200, 201 sind jeweils in einen Kühlkreislauf 500, 501 eingebunden, mit dem jeweils der Festbettreaktor 200, 201 auf einer zulässigen, gekühlten Reaktionstemperatur gehalten wird. In der darstellten und mit vollausgezogenen Linien dargestellten Linien dargestellten Schaltung der Hauptreaktoren 200, 201 wird der erste Hauptreaktor 200 über die Leitung 110 kommend vom Mischbehälter 154 zuerst angeströmt und der erste Reaktionsanteil erfolgt in diesem Hauptreaktor 200. Stromabwärts über einen unteren Auslass und die Leitung 112 wird das Stoffgemisch in den Kopf des zweiten Hauptreaktors 201 geleitet und verlässt diesen über einen Bodenauslass und Leitung 115 in die gemeinsame Leitung 116 als Feed in den gemeinsamen Nachreaktor 210. In die gemeinsame Leitung 116 ist eine Wärmetauscher 206 eingebunden, worüber das für den Nachreaktor 210 erforderliche Temperaturniveau sichergestellt werden kann.

Die Reihenfolge der Durchströmung der beiden Hauptreaktoren kann auch in umgekehrter Reihenfolge vom Hauptreaktor 201 zum Hauptreaktor 200 erfolgen. Hierzu wird in analoger Weise der Hauptreaktor 201 über die Leitung 111 zuerst angeströmt, wobei die Leitung 110 zum Kopf des Hauptreaktors 200 verschlossen ist. Das Stoffgemisch verlässt diesen Festbettreaktor über einen Bodenauslass und die Leitung (Zwischen-)Leitung 113, die mit dem Kopf des Hauptreaktors 200 verbunden ist, wobei die Leitung 115 verschlossen ist. Schließlich verlässt das Stoffgemisch den Hauptreaktor 200 über einen Bodenauslass und Leitung 114, die analog in die gemeinsame Leitung 116 mündet und damit zum Nachreaktor 210 führt. Aus beiden Kühlkreisläufen 500, 501 zweigt jeweils eine Leitung 502, 503 ab, die über jeweils einen Behälter 212, 213 geführt ist, die als Druckausgleichsbehälter dienen, und weiter zu einem Kamin und/oder einer vollständigen Oxidationseinheit. Die Leitungen und Ventile/-einheiten sind derart vorhanden und ausgelegt, dass jeder der Hauptreaktoren 200, 201 alleinige betrieben werden kann und der Stoffstrom um den jeweils anderen Hauptreaktor vollständig vorbeigeleitet werden kann. Die Fließrichtung der beiden Kühlkreisläufe 500, 501 ist durch einen Pfeil symbolisiert, wobei die beide Kühlkreisläufe 500, 501 vorteilhafterweise identisch oder im Wesentlichen gleich ausgebildet sind, da die beiden Hauptreaktoren 200, 201 abwechselnd bzgl. der Strömungsrichtung des Edukt- bzw. des Stoffstroms als erster bzw. zweiter Hauptreaktor betrieben werden.

Die Kühlkreisläufe 500, 501 sind allerdings derart ausgelegt und regelbar, dass je nach verfahrenstechnischen Bedarfen, wie insb. Produktleitung und/oder Produktqualität, jeweils eigenständige Kühlleistungen oder Kühlaufgaben erfüllt werden. Dies betrifft insb. den Volumenstrom pro Zeiteinheit an Kühlmedium und/oder das Temperaturniveau bzw. die zulässige Erwärmung des jeweiligen Kühlmediums. Weiterhin besteht eine direkte Möglichkeit der Wärmeintegration der Kühlkreisläufe 500, 501 mit anderen Analgenabschnitten oder Wärmetauschern, wobei die Wärmetauscher 202, 203 durch die Aufnahme der Reaktionswärme als Wärmequelle dienen, so dass insgesamt der Energieverbrauch der Anlag bzw. des Verfahrens zur Herstellung von PACM gesenkt werden kann.

In der Figur 5 ist weiterhin eine optionale Leitung 117 als gestrichelte Linie (Bypass 2) dargestellt, womit der Nachreaktor 210 umgangen werden kann, wenn beispielsweise dieser gewartet werden muss und/oder die Katalysatorfüllung erneuert werden muss. In diesem Fall wird mind. der in Strömungsrichtung des Stoffstroms zweite Hauptreaktor derart temperiert, dass die vollständige Reaktion bei der gewünschten Produktqualität, insb. dem gewünschten Anteil an den jeweiligen Isomeren sichergestellt ist. Der Leitungsabzweig der Leitung 117 kann in Strömungsrichtung vor oder nach Wärmetauscher 206 vorgesehen werden, wobei es vorteilhaft ist, diesen stromaufwärts zu Wärmetauscher 206 vorzusehen, um diese ggf. auch bypassen zu können und nötige Wartungsarbeiten bei laufender Anlage vornehmen zu können.

Die Umgehung des Hauptreaktors 200 kann im Betrieb des rechts im Bild dargestellten Hauptreaktors 201 über die Leitungen 111, 115 und 116 erfolgen. Die Umgehung des Hauptreaktors 201 kann im Betrieb des links im Bild dargestellten Hauptreaktors 200 über die Leitungen 110, 114 und 116 erfolgen, so dass jeweils die (Kreuz-)Leitungen 112, 113 zwischen den beiden Hauptreaktoren 200, 201 nicht durchflossen werden.

In den Kühlumläufen 500, 501 sind weiterhin (optional) bei der dargestellten Anlagenvariante Wärmetauscher 208, 209 eingebunden. Dieser werden mit einem Heizmedium betrieben, insb. Dampf, und dienen im Anfahrschritt der Hauptreaktoren 200 der (Vor-)Temperierung auf Reaktionstemperatur der jeweiligen Hauptreaktoren 200, 201. Das Niveau dieser Vortemperierung liegt bei ca. 80 bis 100 °C, idealerweise 85 °C bis 95 °C. In dem gezeigten Anlagen- und Verfahrensbeispiel, wie bereits zur Figur 1 ausgeführt, ist es bei dem Ziel, ein möglichst niedriges trans/trans-Isomerenverhältnis von ca. 17 bis 23 Gew.% zur realisieren vorteilhaft, wenn die mit Katalysator neu befüllten Hauptreaktor 200, 201 auf eine Temperatur von ca. 90 °C mittels des jeweiligen Wärmetauschers 208, 209 vorgeheizt werden, bzw. der jeweilige neu befüllte Hauptreaktor 200, 201 entsprechend vorbeheizt wird.

Durch die Vorheizung auf typischerweise 85 bis 95 °C wird ermöglicht, dass die Reaktion bei dem vorliegenden Katalysator unmittelbar starten kann, ohne oder im Wesentlich ohne Recyclingströme des Stoffgemisches der Prozesses bis zum Erreichen der gewünschten Reaktionstemperatur.

Der besondere Vorteil des schaltbaren Hauptreaktors 200, 201 besteht in der Möglichkeit, den zuerst angeströmten Hauptreaktor bei einer höheren Temperatur zu betreiben, weil der Katalysator schon teilweise erschöpft und inaktiviert ist, im Wesentlichen ohne hierbei das trans/trans-Isomerenverhältnis negativ zu beeinflussen (d.h. zu erhöhen). Parallel hierzu fällt stärker erwärmtes Kühlmedium im jeweiligen (Umlauf-)Wärmetauscher 202, 203 des zuerst durchflossenen Hauptreaktors 200, 201 an. Dieses heißere Wärmetauschmedium kann aufgrund der höheren Temperatur besser in der Anlage zur integrierte medienbasierte EK und/oder zum üblichen Wärmetausch mit einem Stoff- Eduktstrom eingesetzt werden.

Die Variante der schaltbaren Hauptreaktoren 200, 201, wie sie in der Figur 6 dargestellt ist, unterscheidet sich zu der gem. Figur 5 darin, dass der Kühlkreislauf 500 des ersten Hauptreaktors 200 mit dem Kühlkreislauf des zweiten Hauptreaktors 201 ebenfalls in Reihe geschaltet ist. Hierbei ist nur ein (kühlender) Wärmetauscher 202 und nur eine Pumpe 204 für die gemeinsame Kühlung und den Medienumlauf vorgesehen, so dass der gemeinsame (zentrale) Leitungsast 508 in beiden Kühlkreisläufen 500, 501 in der Regel ständig und nur in eine Richtung durchströmt wird, unabhängig von der Verschaltung der beiden Hauptreaktoren 200, 201. Hierbei muss der "zentrale" Leitungsast natürlich nicht faktisch zwischen den beiden Hauptreaktoren 200, 201 angeordnet sein. Gezeigt ist die Variante als durchgezogenen Linien, bei der der erste Hauptreaktor 200 (links) zuerst mit dem Eduktgemisch über die Leitung 110 beschickt wird und auch die (Kühlmedien-)Einleitung nach dem Wärmetauscher 202 und der Pumpe 204 zuerst über diesen Hauptreaktor 200 erfolgt. Die in diese Schaltung nicht medienführenden Leitungen oder das Stoffgemisch führenden Leitungen sind gestrichelt dargestellt. Auch bei dieser Varianten besteht die Möglichkeit, das Stoffgemisch und/oder das (Kühl-)Medium vollständig an dem jeweils anderen Hauptreaktor vorbeizuleiten. Somit kann bspw. im Befüllvorgang eines der beiden Hauptreaktoren 200, 201, der jeweils andere unter maximaler Leistung weiter betrieben werden. Der Bypass des Stoffstroms bzw. des jeweiligen Hauptreaktors 200, 201 ergibt sich analog zu Figur 5.

In der gezeigten Beispielschaltung wird der zentrale Leitungsast 508 und der Wärmetauscher 202 durchflossen und über die Leitung 504 im Gleichstrom in den Medienraum ersten Hauptreaktors 200 geleitet, wobei zum gemeinsamen Leitungsknoten führende Leitung 505, kommend vom zweiten Hauptreaktor 201, gesperrt ist. Über die Leitung 506 verlässt das Medium an einem tiefen Auslass den ersten Hauptreaktor 202 und wird zu einem hohen Einlass in den Medienraum des zweiten Reaktors 201 geleitet (Kreuzleitung). Das Medium durchfließt den Medienraum des zweiten Hauptreaktors 201 ebenfalls im Gleichstrom und verlässt diesen an einem tiefliegenden Auslass über die Leitung 509, von der eine Abzweigung wieder in den zentralen Leitungsast 508 mündet, so dass der Kreislauf erneut durchflossen werden kann. Analog wird über die Leitung 505 der rechts dargestellte Hauptreaktor 201 zuerst durchströmt, wenn die Leitung 504 gesperrt ist. Das Medium verlässt dann den Medienraum des zweiten Hauptreaktors 201 über die Leitung 509 und wird an einem hochgelegenen Einlass in den Medienraum des anderen Hauptreaktors 200 geleitet. Der Auslass des Medienraums an einem tiefgelegenen Punkt führt in die Leitung 506 und von dort über eine Abzweigung in den zentralen Leitungsast 508

In der Figur 6 ist analoge zur Figur 5 die optionale Leitung 117 als gestrichelte Linie (Bypass 2) dargestellt, womit der Nachreaktor 210 umgangen werden kann, wobei die Leitung 117 stromabwärts zum (Vorlauf-)Wärmetauscher 206 des Nachreaktors 210 abzweigt.

In der Figur 6 ist weiterhin eine Anlagenvariante (gestrichelt) dargestellt, indem zur Erlangung einer höheren Regelungssicherheit bzw. eines höheren Freiheitsgrades des Temperaturmanagements in der jeweiligen (Kreuz-)Leitung 504, 505 der Kühlkreisläufe 500, 501 bedarfsweise schaltbare und regelbare Wärmetauscher 203 (Vorlaufkühler) angeordnet sind. In dem gezeigten Ausführungsbeispiel sind die beiden Wärmetauscher 202 zur EK in Serie geschaltet, da durch die jeweils inaktive (Kreuz-)Leitung, indem gezeigten Beispiel die Leitung 505, am Einbauort für den Wärmetauscher 202 keine Erwärmung erfolgt. Die Kühlmedienleitung bzw. der Kühlmedienkreislauf der Wärmetauscher 202 kann bei einer vorteilhaften Variante in einem Seitenstrom bzw. über einen Neben- oder Hilfskreislauf über die Pumpe 204 betrieben werden.

Der Vorteil dieses Seitenstroms oder Neben-/Hilfskreislaufes eines Kühlmittels über die Wärmetauscher 203 hat den großen Vorteil, dass diese zusätzliche Kühlleistung nur bedarfsweise abgerufen werden braucht und mit geringem Aufwand eine weitergehend, bedarfsmäßige Regelung der Temperatur im jeweils zweiten beiden seriell geschalteten Hauptreaktoren erfolgen kann.

Der (Vorlauf-)Wärmetauscher 206 ist vorliegend vorrangig beschrieben und teilweise in einer EK gezeigt, bei denen dieser als Wärmetauscher 206 vorrangig als Wärmesenke arbeitet, also der darin geführte Stoffstrom erwärmt wird. Durch die an den Hauptreaktor 200, 201 angepasste, abhängige Betriebsweise des Nachreaktors 210 kann mind. zeitweise, insb. dauerhaft, eine Kühlung des Stoffstroms in der (Feed-)Leitung 116 stromaufwärts zum Nachreaktor 210 erforderlich sein, weil im (adiabatischen) Nachreaktor ca. 10 bis 20% des Umsatzes erfolgen, so dass eine Erwärmung des Stoffstroms gemessen im Auslass bis auf ca. 140 °C erfolgt. Somit kann unabhängig von den hierhin beschriebenen Ausführungsformen und Varianten der Anlagen und des Verfahrens eine angepasste EK zur Kühlung (Wärmetauscher 206 arbeitet als Wärmequelle) vorgesehen werden. Alternativ oder zusätzlich kann eine ergänzende Kühlung über eine geänderte oder weitere EK vorgesehen werden.

Insgesamt sind eine Vielzahl von üblichen, dem Fachmann bekannte und für eine vorteilhafte Prozessführung nötige oder sinnvolle Steuer- und Regelungselemente nicht dargestellt, wie Sensoren (Durchfluss, Temperatur, Druck etc.), Anzeigen, Stell- und Regelglieder (insb. Ventile, weitere Pumpen, Verdichter), Sammelbehälter etc. und sind bedarfsweise zu ergänzen. Insb. sind auch bei der Nennung von "einer" Pumpe oder "einem" Verdichter übliche Redundanzen aus mind. zwei parallelen Aggregaten gemeint, insb. von zwei parallelen Pumpen oder zwei parallelen Verdichtern. In analoger Weise ist "ein Wärmetauscher" nicht limitierend zu verstehen und meint mit der jeweiligen, örtlichen Wärmetauschaufgabe auch Anordnungen von in Reihe geschalteten Wärmetauschern oder parallel geschaltete, auch redundanten, Wärmetauschern, wobei hiermit keine Verschaltungen mit mind. einem weiteren Wärmetauscher und örtlich unterschiedlicher Wärmetauschaufgabe zu verstehen ist.

Grundsätzlich sind alle Wärmetauscher und Kondensatoren derart ausgebildet, dass ein indirekter Wärmeübertrag erfolgt und keine stoffliche Vermischung von Edukten, Produkt, Nebenprodukten und/oder Lösungsmittel mit den (Heiz-/Kühl-)Medium, wie Gas, Dampf, Wasser, Öl, Sole (Brine) etc. erfolgt, es sei denn es ist etwas hiervon Abweichendes ausgeführt.

Auch wenn vorliegend Bauteile, wie Ventile, Druckregeleinheit, Absperraggregate etc. zur vereinfachten Beschreibung einzeln oder gesondert dargestellt sind und zum Teil nicht einzeln genannt wurde, ist dies nicht limitierend zu lesen, vielmehr kann der Fachmann bedarfsweise zwei oder mehrere dieser Bauteile in einer Ventil- oder Stelleinheit zusammenfassen oder Mehrwegventile stattdessen vorsehen.

Vorliegend meint "stromaufwärts" oder "stromabwärts" die Anordnung und/oder Fließrichtung des produktreichen Stoffstroms, wenn nichts hiervon Abweichendes ausgeführt ist. Weiterhin ist unter "Medien", "Medienstrom", "Medienleitung" etc. immer ein Heiz- oder Kühlmedium bzw. die zugehörige Leitung gemeint, wenn nichts hiervon Abweichendes ausgeführt ist.

Für alle Ausführungsformen und Varianten der Anlage und des Verfahrens kann generell vorgesehen werden, dass die zweite Trennkolonne 340 und die dritte Trennkolonne 350 als eine einzige Kolonne ausgeführt werden, insb. als eine Trennwandkolonne (nicht dargestellt). Hierbei können vorteilhafterweise die Trennaufgaben der zweite Trennkolonne 340 und der dritten Trennkolonne 350 mind. teilweise, idealerweise vollständig mittels der Trennwandkolonne (nicht dargestellt) ausgeführt werden, wie sie bspw. aus den Druckschriften EP 012 62 88 B1 oder EP 012 23 67 A2 bekannt sind.

Insgesamt kann ein großer, energetischer Vorteil mit der erfindungsgemäßen Anlage und dem Verfahren erreicht werden, der darin besteht, dass eine starke Verringerung der extern zugeführten Energieströme ermöglicht wurde, insb. die Einsparung von großen Mengen an (externem) Heizdampf.

## Patentansprüche

1. Anlage (100) zur kontinuierlichen, katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2),
umfassend eine Konditionierungseinheit (104) für die Edukte, eine Reaktoreinheit (102) zur Synthese von PACM und eine Trenneinheit (106), wobei
- die Konditionierungseinheit (104) (Zu-)Leitungen für Edukte1, Edukt2 und mind. ein Lösungsmittel, mind. einen Wärmetauscher (158) in mind. einer (Zu-)Leitung, mind. einen Mischer (152) zur Mischung der Edukte und/oder mind. eines Edukts mit mind. einem Lösungsmittel umfasst;
- die Reaktoreinheit (102) mind. einen Festbettreaktor als Hauptreaktor (200, 201) mit einer immobilen Katalysatorpackung umfasst, wobei der mind. eine (erste) Hauptreaktor (200, 201)
- einen ersten Strömungsweg für das Stoffgemisch
über die immobile Katalysatorpackung und
- einen weiteren hiervon getrennten, geschlossenen Strömungsweg für ein Wärmetauschmedium außerhalb der Katalysatorpackung umfasst, und
wobei in den Medienumlauf ein Wärmetauscher (202) eingebunden ist;
- die Trenneinheit (106) mind.
- eine erste Trennstufe (106A) zur (im Wesentlichen) Abtrennung des Lösungsmittels und
- eine zweite Trennstufe (106B) zur Abtrennung vom mind. einem Edukt und/oder mind.
einem Nebenprodukt vom Produkt umfasst,
**dadurch gekennzeichnet, dass**
die Trenneinheit (106) in der ersten Trennstufe (106A) mind. ein Druckregeleinheit (220), mind. einen Trennkessel (300) und eine Kondensationseinheit (304) für das Lösungsmittel umfasst, wobei eine (Rück-)Leitung (311) für das Lösungsmittel von der mind. einen Kondensationseinheit (304) der ersten Trennstufe zur Konditionierungseinheit (104) führt.

2. Anlage nach Anspruch 1, **dadurch gekennzeichnet, dass**
die Reaktoreinheit einen ersten Hauptreaktor (200) und mind. einen stromabwärts befindlichen permanenten, nicht schaltbaren weiteren Nachreaktor (210) umfasst.

3. Anlage nach Anspruch 1 oder 2, **dadurch gekennzeichnet, dass** die Reaktoreinheit (106) einen weiteren Hauptreaktor (201) als Festbettreaktor umfasst, der
- einen ersten Strömungsweg für das Stoffgemisch und
- einen weiteren (geschlossenen) Strömungsweg für ein Wärmetauschmedium umfasst, und wobei stromaufwärts zu den beiden Hauptreaktoren (200, 201) in der (Zu-)Leitung (110, 111) eine Ventileinheit vorgesehen ist, mittels welcher der Volumenstrom der Eduktmischung zw. dem ersten Hauptreaktor (200) und dem weiteren Hauptreaktor (201) aufteilbar, durchleitbar und/oder vollständig umschaltbar ist, und wobei beide Hauptreaktoren (200, 201)
- jeweils mit einem Wärmetauscher (202, 203 oder
- mit einem gemeinsamen Wärmetauscher (202)
für den weiteren (geschlossenen) Strömungsweg verbunden sind.

4. Anlage nach Vorrichtungsanspruch 2 oder 3, **dadurch gekennzeichnet, dass** in der Leitung (116) zwischen dem mind. einen Hauptreaktor (200, 201) und dem Nachreaktor (210) mind. ein Wärmetauscher (206) angeordnet ist.

5. Anlage nach einem der vorherigen Vorrichtungsansprüche, **dadurch gekennzeichnet, dass** die Trenneinheit einen Trennkessel (300) umfasst der mit dem Hauptreaktor (200) oder dem Nachreaktor (210) über eine Leitung (211) verbunden ist, insb. dem in Strömungsrichtung letzten Reaktor (200, 201, 210) verbunden ist, wobei der Trennkessel (300)
- einen Kopfauslass,
- einen Boden-/Sumpfauslass und
- einen beheizbaren Sumpfumlauf mit mind. einem Wärmetauscher (302) umfasst, wobei in die (Kopf-)Leitung (163) eine Kondensationseinheit (304) eingebunden ist, und wobei stromabwärts der Kondensationseinheit (304)
- ein (Sammel-)Behälter (310) und/oder
- eine Verbindungseinheit/-knoten mit/in die (Rück-)Leitung (311) für das Lösungsmittel angeordnet ist.

6. Anlage nach einem der vorherigen Vorrichtungsansprüche, **dadurch gekennzeichnet, dass** die Trenneinheit einen zweiten Trennkessel (301) umfasst, der stromabwärts zum ersten Trennkessel (300) angeordnet ist, wobei der Sumpfauslass des ersten Trennkessels (300) mit dem Einlass des zweiten Trennkessels (301) verbunden ist, wobei der zweite Trennkessel (301) einen Kopfauslass, einen Boden-/Sumpfauslass und einen beheizbaren Sumpfumlauf mit mind. einem Wärmetauscher (303) umfasst, wobei eine (Kopf-)Leitung von dem (Kopf-)Auslass des zweiten Trennkessels (301) zu einer Kondensationseinheit (304, 305, 315) und/oder in die (Kopf-)Leitung (162, 163) des ersten Kessels (300) führt.

7. Anlage nach Vorrichtungsanspruch 6, **dadurch gekennzeichnet, dass** die Kondensationseinheit (304) mind. zwei Wärmetauscher (305, 315) umfasst, wobei zwischen den beiden Wärmetauscher (305, 315) der Kondensationseinheit (304) die Verbindungseinheit/-knoten für die (Kopf-)Leitung des zweiten Kessels (301) mit der (KopfLeitung (163) des ersten Kessels (300) angeordnet ist.

8. Anlage nach Vorrichtungsanspruch 6 oder 7, **dadurch gekennzeichnet, dass** eine (MedienLeitung (510) vom Ausgang der Kondensationseinheit (304), insb. mind. einer der Wärmetauscher (305, 315), zum Eingang des Wärmetauschers (303) des zweiten Trennkessels (301) führt.

9. Anlage nach einem der Vorrichtungsansprüche 1 bis 5, **dadurch gekennzeichnet, dass** die Trenneinheit in der ersten Trennstufe als Trennkessel mind. zwei in Reihe geschaltete Verdampfer (370, 380) umfasst, wobei in der (Kopf-)Leitung (162) mind. eines der beiden Verdampfers (370, 380) als Kondensationseinheit (304) mind. ein Wärmetauscher (315) vorgesehen ist, und wobei stromabwärts des Wärmetauschers (315) in der (Kopf-)Leitung (162)
- ein (Sammel-)Behälter (310)) und/oder
- eine Verbindungseinheit/-knoten mit/in die (Rück-)Leitung (311) für das Lösungsmittel angeordnet ist.

10. Anlage nach Vorrichtungsanspruch 9, **dadurch gekennzeichnet, dass** mind. einer der Verdampfer (370, 380) oder alle Verdampfer (370, 380) als so genannte Kettle-Type-Verdampfer ausgebildet sind.

11. Anlage nach Vorrichtungsanspruch 9 oder 10, **dadurch gekennzeichnet, dass**
- das WT-Teil (376) des ersten Verdampfers (370) mit einer Medienleitung verbunden ist, und wobei die Leitungsführung zwischen den Verdampfern (370, 380) derart ist, dass der PACM-reiche Stoffstrom als Hochsiedeanteil im Sumpf der beiden Verdampfer (370, 380) geführt wird.

12. Anlage nach Vorrichtungsanspruch 9 bis 11, **dadurch gekennzeichnet, dass** eine (Stoff-Leitung (163) für den lösungsmittelreichen Stoffstrom vom (Kopf-)Auslass (372) des ersten Verdampfers (370) zum Einlass (383) des WT-Teils (386) des zweiten Verdampfers (380) führt, und wobei einen Leitung (161) vom die Sumpfauslass (385) zu
- einem (Sammel-)Behälter (120) und/oder
- einer Verbindungseinheit/-knoten mit/in die (Rück-)Leitung (311) für das Lösungsmittel führt.

13. Verfahren zur katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2), wobei die Herstellung mittels einer industriellen Anlage (100) erfolgt, **dadurch gekennzeichnet, dass**
die Anlage (100) nach mindestens einem der vorstehenden Vorrichtungsansprüche ausgebildet ist.

14. Verfahren nach Anspruch 13, **dadurch gekennzeichnet, dass** die Temperatur des Eduktstroms am Einlass des Hauptreaktors (200, 201) 80 bis 135°C beträgt, idealerweise 90 bis 135 °C.

15. Verfahren nach einem der vorherigen Verfahrensansprüche, **dadurch gekennzeichnet, dass** der Druck im Hauptreaktor (200, 201) 60 bis 120 bar beträgt, idealerweise 70 bis 110 bar.

16. Verfahren nach einem der vorherigen Verfahrensansprüche, **dadurch gekennzeichnet, dass** dieses kontinuierlich, katalytisch zur Produktion von Methylenbis(cyclohexylamin) erfolgt, insb. zur Produktion von 4,4'-Diaminodicyclohexylmethan (PACM), bevorzugt 4,4'-Diaminodicyclohexylmethan (PACM) mit niedrigen Anteilen an trans/trans-Isomeren.

17. Verfahren nach einem der vorherigen Verfahrensansprüche, **dadurch gekennzeichnet, dass**
- die Temperatur am Eingang des Hauptreaktors (200, 201) im Wesentlichen der Temperatur am Eingang des Nachreaktors (210) entspricht, wobei im Wesentlichen einen Bereich oder Unterschied von +/- 10 °C meint und/oder
der Druck am Eingang des Hauptreaktors (200, 201) im Wesentlichen dem Druck am Eingang des Nachreaktors (210) entspricht, wobei im Wesentlichen einen Bereich oder Unterschied von +/- 5 bar meint.

18. Verfahren nach einem der vorherigen Verfahrensansprüche, **dadurch gekennzeichnet, dass** das MDA (Edukt1) eine Mischung der folgenden Monomere umfasst: 4,4' MDA, 2,4' MDA und 2,2' MDA, wobei der Anteil an 4,4' MDA vorteilhafterweise im Bereich von 75 bis 98 mol-% beträgt, idealerweise 85 bis 95 mol-%.

19. Verfahren nach einem der vorherigen Verfahrensansprüche, **dadurch gekennzeichnet, dass** die erste Trennstufe (106A) der Trenneinheit (106) zum (ersten) Trennkessel (300) mind. einen weiteren (zweiten) Trennkessel (301) umfasst, der stromabwärts zum ersten Trennkessel (300) angeordnet ist, wobei ein Sumpfauslass des ersten Trennkessels (300) mit einem Einlass des zweiten Trennkessels (301) verbunden ist, wobei der erste Trennkessel (300) bei einer ersten Temperatur und einem ersten Druck betreiben wird und der zweite Trennkessel (301) bei einer zur ersten Temperatur tieferen zweiten Temperatur und einem gegenüber dem ersten Druck niedrigeren Druck betrieben wird.
